# EUROPEAN PATENT SPECIFICATION

(11) **EP 2 508 116 B1**
(45) Date of publication and mention of the grant of the patent: **13.11.2013**
(21) Application number: 11826617.0
(22) Date of filing: 22.06.2011
(51) Int. Cl.: A61B 1/00, A61B 1/04, G06F 19/00, G06F 17/18

(54) **IMAGE-DISPLAY DEVICE AND CAPSULE-TYPE ENDOSCOPE SYSTEM**
BILDANZEIGEVORRICHTUNG UND KAPSELARTIGES ENDOSKOPSYSTEM
DISPOSITIF D'AFFICHAGE D'IMAGE, ET SYSTÈME D'ENDOSCOPE DE TYPE CAPSULE

(30) Priority: 24.09.2010 JP 2010214413
(43) Date of publication of application: 10.10.2012
(73) Proprietor: OLYMPUS MEDICAL SYSTEMS CORP., Tokyo 151-0072 (JP)
(72) Inventor: KOBAYASHI, Satomi, Tokyo 151-0072 (JP); TAKASUGI, Kei, Tokyo 151-0072 (JP)
(74) Representative: Gunzelmann, Rainer
(86) International application number: PCT/JP2011/064260
(87) International publication number: WO 2012/039171

(56) References cited:
- JP-A- 2007 283 001
- JP-A- 2010 082 241
- US-A1- 2002 051 570
- US-A1- 2007 053 614

## Description

### TECHNICAL FIELD

The present invention relates to an image display apparatus and a capsule endoscope system for displaying an in-vivo image that is obtained by a capsule endoscope introduced into the subject.

### BACKGROUND ART

Conventionally, in diagnosis of a subject using a capsule endoscope introduced into the subject and capturing an image in the subject, operation is performed such that a group of in-vivo images obtained by the capsule endoscope is observed as a quasi-motion picture or as a list of still pictures, and those appearing to be abnormal are selected therefrom. This operation is called interpretation of radiogram.

The group of in-vivo images captured in one examination includes as many as approximately 60,000 images (equivalent to about 8 hours), and therefore, extremely heavy burden is imposed on a person who does interpretation of radiograms. For this reason, as a helping function for the interpretation of radiograms, it is suggested to, e.g., detect an abnormal portion such as bleeding and tumor by image processing and draw the trace of the capsule endoscope by estimating, through calculation, the position where an in-vivo image in question is captured in the subject (see, for example, patent literatures 1 to 3).

### Citation List

### Patent Literature

Patent Literature 1: Japanese Patent Application Laid-open No. 2008-36243
Patent Literature 2: Japanese Patent Application Laid-open No. 2010-82241
Patent Literature 3: Japanese Patent Application Laid-open No. 2007-283001

### DISCLOSURE OF INVENTION

### PROBLEM TO BE SOLVED BY THE INVENTION

Because, as described above, many in-vivo images are captured in one examination, if the above helping function is performed on every in-vivo image, it is necessary to process a large amount of data. It is noted that the precision and the range necessary for a helping function differ depending on the type of an examination, the skill of a person who does interpretation of radiograms, etc. Therefore, if the helping function is performed in a single uniform way, for a given examination, because of processing of data whose amount is larger than necessary, a longer time is needed. This delays the time to start interpretation of radiograms and, thus, such a manner is inefficient.

The present invention has been achieved to solve the above problem and it is an object of the present invention to provide an image display apparatus and a capsule endoscope system enabling an efficient interpretation of radiograms regarding image data captured by a capsule endoscope.

### MEANS FOR SOLVING PROBLEM

To solve the above problem and achieve the above object, the present invention provides a capsule endoscope system as defined in claim 1. Said capsule endoscope system comprises an image display apparatus as defined in the claim.

In the abovementioned image display apparatus, the processing setting unit may cause the image processing and the position estimating processing to be executed in parallel, and the processing time may be actual processing time in which the image processing and the position estimating processing are executed in parallel.

In the abovementioned image display apparatus, the image processing unit can execute at least one of red color detecting processing and detecting processing of a predetermined lesion site.

In the abovementioned image display apparatus, the processing setting unit may set precision of the image processing executed by the image processing unit.

The abovementioned image display apparatus may further include a trace calculation unit for calculating a trace of the capsule endoscope in the subject, on the basis of execution result of the position estimating processing by the position estimating unit.

In the abovementioned image display apparatus, the input receiving unit may receive an input of a desired processing time or a desired finish time of the processing by the image processing unit and/or the position estimating unit. The processing setting unit may generate a processing content serving as a candidate of a position estimation level of the position estimating processing executed by the position estimating unit and/or the content of the image processing executed by the image processing unit, on the basis of the desired processing time or the desired finish time. The display unit may display the processing content generated by the processing setting unit.

In the abovementioned image display apparatus, the processing setting unit may generate a processing content in which the image processing and/or the position estimating processing can be finished within a predetermined range from the desired processing time or the desired finish time.

In the abovementioned image display apparatus, the input receiving unit may receive an input of a desired processing time or a desired finish time for the processing by the image processing unit and/or the position estimating unit. The processing setting unit may generate a plurality of processing contents serving as candidates of position estimation levels of the position estimating processing executed by the position estimating unit and/or the content of the image processing executed by the image processing unit, on the basis of the desired processing time or the desired finish time. The display unit may display the plurality of processing contents. When the input receiving unit receives an input of a selection signal for selecting one of the plurality of processing contents, the processing setting unit may set image processing included in the selected processing content as the processing executed by the image processing unit, and may set a position estimating processing at a position estimation level included in the selected processing content as the processing executed by the position estimating unit.

In the abovementioned image display apparatus, the input receiving unit may receive an input of a desired processing time or a desired finish time of the processing by the image processing unit and/or the position estimating unit, and may receive an input of an order of priority of the image processing and/or the position estimating processing The processing setting unit may generate a processing content serving as a candidate of a position estimation level of the position estimating processing executed by the position estimating unit and/or the content of the image processing executed by the image processing unit, on the basis of the desired processing time or the desired finish time and the order of priority. The display unit may display the processing content generated by the processing time estimating unit.

In the abovementioned image display apparatus, the processing time estimating unit may respectively predict individual processing times required by the position estimating processing at respective position estimation levels that can be executed by the position estimating unit and various kinds of image processing that can be executed by the image processing unit. The display unit may display the individual processing times predicted by the processing time estimating unit in association with the various kinds of image processing and the respective position estimation levels.

In the abovementioned image display apparatus, the input receiving unit may receive an input of selection information for selecting a portion of the subject on which the image processing and/or the position estimating processing are performed. The processing setting unit may set a range of the in-vivo image data to be subjected to the processing performed by the image processing unit and/or the position estimation, on the basis of the selection information.

### EFFECT OF THE INVENTION

With the present invention, for in-vivo image data captured by a capsule endoscope, the content of image processing and a position estimation level are set on the basis of information received by an input receiving unit and a time required for these processes is predicted and displayed; therefore, an efficient interpretation of radiograms is enabled of in-vivo images that are subjected to a necessary process.

### BRIEF DESCRIPTION OF DRAWINGS

[Fig. 1] FIG. 1 is a schematic diagram illustrating a schematic configuration of a capsule endoscope system according to a first embodiment of the present invention.
[Fig. 2] FIG. 2 is a schematic diagram illustrating a schematic configuration of a capsule endoscope as illustrated in FIG. 1.
[Fig. 3] FIG. 3 is a block diagram illustrating a configuration of a receiving device and the capsule endoscope as illustrated in FIG. 1.
[Fig. 4] FIG. 4 is a block diagram illustrating a configuration of an image display apparatus as illustrated in FIG. 1.
[Fig. 5] FIG. 5 is a block diagram illustrating a detailed configuration of an image processing unit.
[Fig. 6] FIG. 6 is a flowchart illustrating operation of the image display apparatus as illustrated in FIG. 4.
[Fig. 7] FIG. 7 is a schematic diagram illustrating an example of a processing selection screen displayed in the first embodiment.
[Fig. 8] FIG. 8 is a schematic diagram illustrating an example of a processing selection screen displayed in the first embodiment.
[Fig. 9] FIG. 9 is a schematic diagram illustrating an example of a radiographic image interpretation screen displayed in the first embodiment.
[Fig. 10] FIG. 10 is a schematic diagram illustrating an example of a processing selection screen displayed in a first modification of the image display apparatus according to the first embodiment.
[Fig. 11] FIG. 11 is a block diagram illustrating a configuration of a second modification of the image display apparatus according to the first embodiment.
[Fig. 12] FIG. 12 is a schematic diagram illustrating an example of a radiographic image interpretation screen displayed in the second modification of the image display apparatus according to the first embodiment.
[Fig. 13] FIG. 13 is a flowchart illustrating operation of the image display apparatus according to a second embodiment of the present invention.
[Fig. 14] FIG. 14 is a schematic diagram illustrating an example of a processing selection screen displayed in the second embodiment.
[Fig. 15] FIG. 15 is a figure illustrating an example of a processing content table generated in the second embodiment.
[Fig. 16] FIG. 16 is a schematic diagram illustrating an example of processing time input field displayed on a display unit.
[Fig. 17] FIG. 17 is a schematic diagram illustrating the processing time input field into which a desired processing time is input.
[Fig. 18] FIG. 18 is a schematic diagram illustrating an example of a processing selection screen displayed in the second embodiment.
[Fig. 19] FIG. 19 is a schematic diagram illustrating an example of a processing candidate display screen displayed in a first modification of the image display apparatus according to the second embodiment.
[Fig. 20] FIG. 20 is a figure illustrating an example of a processing content table generated in the second modification of the image display apparatus according to the second embodiment.
[Fig. 21] FIG. 21 is a schematic diagram illustrating an example of a priority order setting screen displayed in a third modification of the image display apparatus according to the second embodiment.
[Fig. 22] FIG. 22 is a schematic diagram illustrating an example of a precision setting screen displayed in a third modification of the image display apparatus according to the second embodiment.
[Fig. 23] FIG. 23 is a figure illustrating a search method of a processing content executed in the third modification of the image display apparatus according to the second embodiment.
[Fig. 24] FIG. 24 is a figure for illustrating a priority order method executed in a fourth modification of the image display apparatus according to the second embodiment.
[Fig. 25] FIG. 25 is a schematic diagram illustrating an example of a processing selection screen displayed in the third embodiment.
[Fig. 26] FIG. 26 is a schematic diagram illustrating an example of a processing selection screen displayed in the third embodiment.
[Fig. 27] FIG. 27 is a schematic diagram illustrating an example of a processing selection screen displayed in the third embodiment.
[Fig. 28] FIG. 28 is a schematic diagram illustrating an example of a processing selection screen displayed in the third embodiment.
[Fig. 29] FIG. 29 is a schematic diagram illustrating an example of a processing selection screen displayed in a modification of the image display apparatus according to the third embodiment.
[Fig. 30] FIG. 30 is a schematic diagram illustrating an example of a processing selection screen displayed in the image display apparatus according to a fourth embodiment.
[Fig. 31] FIG. 31 is a schematic diagram illustrating an example of a processing selection screen displayed in a modification of the image display apparatus according to the fourth embodiment.

### BEST MODE(S) FOR CARRYING OUT THE INVENTION

Hereinafter, a capsule endoscope system according to an embodiment of the present invention will be described with reference to drawings. In the following description, for example, a system including a capsule endoscope for capturing an in-vivo image introduced into a subject will be illustrated as an example, but it is to be understood that the present invention is not limited by this embodiment.

### (First embodiment)

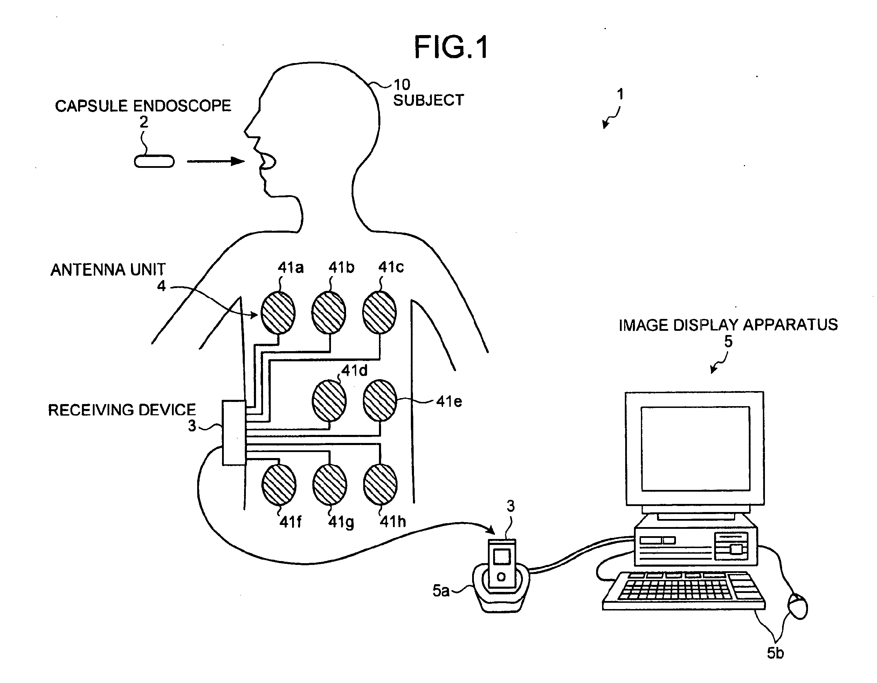

FIG. 1 is a schematic diagram illustrating a schematic configuration of a capsule endoscope system according to a first embodiment of the present invention. This capsule endoscope system 1 includes a capsule endoscope 2 being introduced into a subject 10 to capture an image and wirelessly transmitting image data of the in-vivo image to a receiving device 3, the receiving device 3 for receiving the in-vivo image data wirelessly transmitted from the capsule endoscope 2, and an image display apparatus 5 for displaying, on a screen, the in-vivo image based on the in-vivo image data received by the receiving device 3.

After the capsule endoscope 2 is swallowed through the mouth of the subject 10, the capsule endoscope 2 moves in organs of the subject 10 due to peristaltic motions of organs and the like, and during which the capsule endoscope 2 generates the in-vivo image data by performing predetermined signal processing on captured image signals obtained by successively capturing images in the subject 10 with a predetermined time interval (for example, interval of 0.5 seconds). Every time the capsule endoscope 2 captures the in-vivo image of the subject 10, the capsule endoscope 2 successively, wirelessly transmits the generated in-vivo image data to the external receiving device 3. Identification information (for example, serial number) for identifying an individual capsule endoscope is allocated to the capsule endoscope 2, and this identification information is wirelessly transmitted together with the in-vivo image data.

The receiving device 3 has an antenna unit 4 having multiple receiving antennas 41a to 41h. Each of the receiving antennas 41a to 41h is configured using, for example, a loop antenna, and each of the receiving antennas 41a to 41h is provided at a predetermined position on an external surface of the subject 10 (for example, a position corresponding to each organ in the subject 10, which is a path of the capsule endoscope 2). These receiving antennas 41a to 41h are provided at, for example, the predetermined positions with respect to the subject 10 during the examination. It should be noted that the arrangement of the receiving antennas 41a to 41h may be changed to any arrangement in accordance with the purpose of examination, diagnosis, and the like. It should be noted that the number of antennas provided in the antenna unit 4 may not be necessarily interpreted as being eight, which are shown as the receiving antennas 41a to 41h, and the number of antennas provided in the antenna unit 4 may be less than eight or may be more than eight.

While the capsule endoscope 2 captures images (for example, from when the capsule endoscope 2 is introduced through the mouth of the subject 10 to when the capsule endoscope 2 passes through the alimentary canal and is excreted), the receiving device 3 is carried by the subject 10, and receives, via the antenna unit 4, the in-vivo image data wirelessly transmitted from the capsule endoscope 2. The receiving device 3 stores the received in-vivo image data to memory incorporated therein. The receiving device 3 also stores, to the memory, received strength information about each of the receiving antennas 41a to 41h when the in-vivo image is received and time information representing a time at which the in-vivo image is received, in such a manner that the received strength information and the time information are associated with the in-vivo image data. It should be noted that the received strength information and the time information are used by the image display apparatus 5 as information related to the position of the capsule endoscope 2. After the capsule endoscope 2 finishes capturing the images, the receiving device 3 is detached from the subject 10, and is connected to the image display apparatus 5 so that information such as the in-vivo image data is transferred (downloaded).

The image display apparatus 5 is configured with a work station or a personal computer having a display unit such as a CRT display or a liquid crystal display, and displays the in-vivo image based on the in-vivo image data obtained via the receiving device 3. An operation input device 5b such as a keyboard and a mouse is connected to the image display apparatus 5. Alternatively, a touch panel provided in an overlapping manner on the display unit may be used as the operation input device 5b. While a user (a person who does interpretation of radiograms) manipulates the operation input device 5b, the user interprets the in-vivo images of the subject 10 which are displayed successively on the image display apparatus 5, and observes (examines) living body portions (for example, esophagus, stomach, small intestine, and large intestine) in the subject 10, thus diagnosing the subject 10 on the basis of the above.

The image display apparatus 5 has, for example, a USB (Universal Serial Bus) port, and a cradle 5a is connected via this USB port. The cradle 5a is a reading device for reading the in-vivo image data from the memory of the receiving device 3. When the receiving device 3 is attached to the cradle 5a, the receiving device 3 is electrically connected to the image display apparatus 5, so that the in-vivo image data stored in the memory of the receiving device 3, the received strength information and the time information associated therewith, and the related information such as the identification information of the capsule endoscope 2 are transferred to the image display apparatus 5. The image display apparatus 5 thus obtains the series of the in-vivo image data of the subject 10 and the related information related thereto, and further executes processing explained later, thus displaying the in-vivo image. It should be noted that the image display apparatus 5 may be connected to an output device such as a printer, and the in-vivo image may be output to the output device.

It should be noted that the image display apparatus 5 can obtain the in-vivo image data captured by the capsule endoscope 2 according to various types of methods other than the method explained above. For example, in the receiving device 3, memory that can be detached from and attached to the receiving device 3, such as a USB memory and a compact flash (registered trademark), may be used instead of the internal memory. In this case, after the in-vivo image data provided by the capsule endoscope 2 are stored to the memory, only the memory may be detached from the receiving device 3, and, for example, the memory may be inserted into the USB port and the like of the image display apparatus 5. Alternatively, the image display apparatus 5 may be provided with a communication function for communicating with an external device, and the in-vivo image data may be obtained from the receiving device 3 by means of wired or wireless communication.

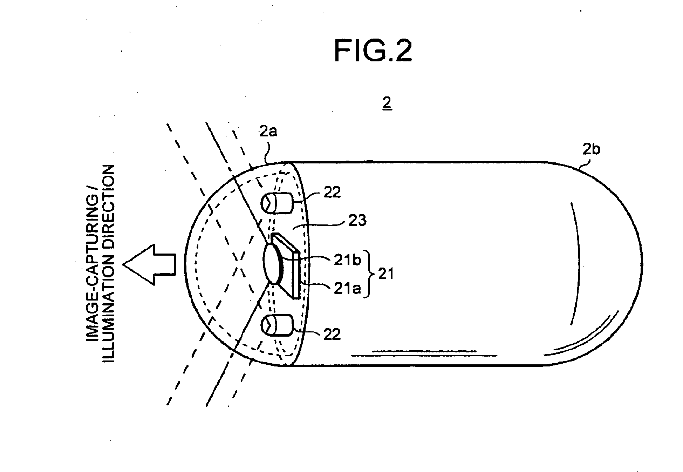

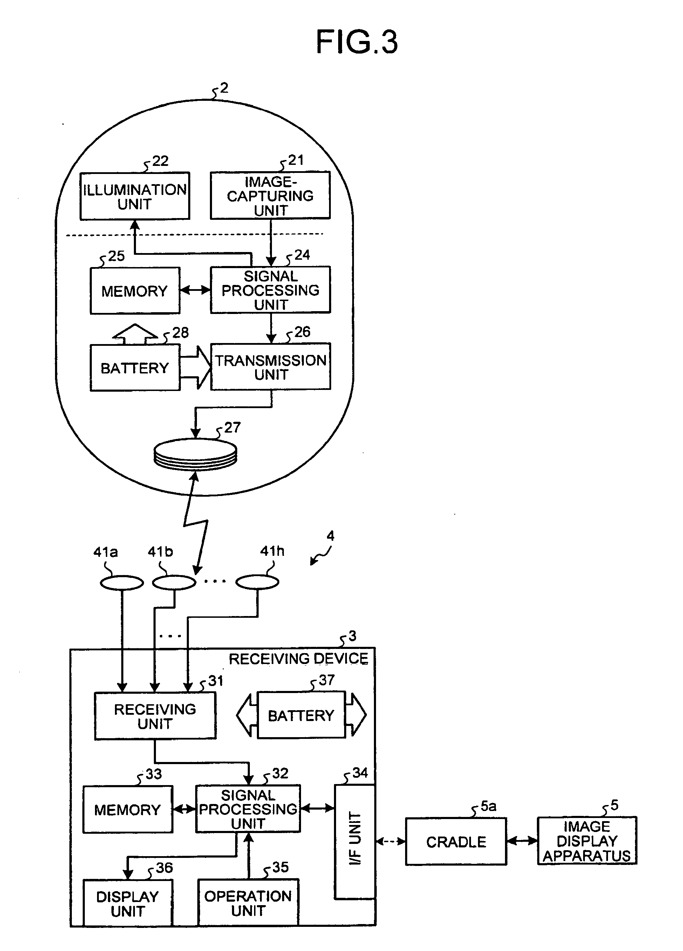

Subsequently, each device constituting the capsule endoscope system 1 will be explained in detail. FIG. 2 is a schematic diagram illustrating an example of configuration of the capsule endoscope 2. FIG. 3 is a block diagram illustrating a configuration of the capsule endoscope 2 and the receiving device 3.

As illustrated in FIG. 2, the capsule endoscope 2 is housed in a capsule-shaped container (casing) including a container 2b in a substantially cylindrical shape or a semi-elliptically spherical shape in which one end is a hemispherical dome shape and the other end is open and a hemispherical optical dome 2a for sealing the container 2b in a watertight manner when the optical dome 2a is fastened to the opening of the container 2b. The capsule-shaped container (2a, 2b) is of a size such that, for example, the subject 10 can swallow the capsule- shaped container (2a, 2b). In the present embodiment, at least the optical dome 2a is made of a transparent material.

As illustrated in FIGS. 2 and 3, the capsule endoscope 2 includes an image-capturing unit 21 for capturing an image inside of the subject 10, an illumination unit 22 for illuminating the inside of the subject 10 during image capturing process, a circuit board 23 formed with a drive circuit and the like for respectively driving the image-capturing unit 21 and the illumination unit 22, a signal processing unit 24, memory 25, a transmission unit 26, an antenna 27, and a battery 28.

The image-capturing unit 21 includes, for example, an image sensor 21a such as a CCD and a CMOS for generating image data of an image of the subject from an optical image formed on a light receiving surface, and also includes an optical system 21b such as an object lens provided at the light receiving surface side of the image sensor 21a. The illumination unit 22 is configured with an LED (Light Emitting Diode) and the like for emitting light to the subject 10 during the image capturing process. The image sensor 21a, the optical system 21b, and the illumination unit 22 are mounted on the circuit board 23.

The drive circuit of the image-capturing unit 21 operates under the control of the signal processing unit 24 explained later, and generates, for example, a captured image signal representing an image in the subject with a regular interval (for example, two frames per second), and the captured image signal is input to the signal processing unit 24. In the explanation below, it is assumed that the image-capturing unit 21 and the illumination unit 22 respectively include their drive circuits.

The circuit board 23 having the image-capturing unit 21 and the illumination unit 22 mounted thereon is provided at the side of the optical dome 2a within the capsule- shaped container (2a, 2b) such that the light receiving surface of the image sensor 21a and the light emitting direction of the illumination unit 22 face the subject 10 with the optical dome 2a interposed therebetween. Therefore, the image capturing direction of the image-capturing unit 21 and the illumination direction of the illumination unit 22 are oriented toward the outside of the capsule endoscope 2 with the optical dome 2a interposed therebetween as illustrated in FIG. 2. Accordingly, while the illumination unit 22 illuminates the inside of the subject 10, the image-capturing unit 21 can capture images in the subject 10.

The signal processing unit 24 controls each unit in the capsule endoscope 2, and also performs A/D conversion on the captured image signal that is output from the image-capturing unit 21 to generate digital in-vivo image data, and further performs predetermined signal processing. The memory 25 temporarily stores various types of operations executed by the signal processing unit 24 and the in-vivo image data having been subjected to signal processing by the signal processing unit 24. The transmission unit 26 and the antenna 27 transmits, to the outside, the in-vivo image data stored in the memory 25 as well as the identification information of the capsule endoscope 2 in such a manner that the in-vivo image data and the identification information are multiplexed in a radio signal. The battery 28 provides electric power to each unit in the capsule endoscope 2. It is assumed that the battery 28 includes a power supply circuit for, e.g., boosting the electric power supplied from a primary battery such as a button battery or a secondary battery.

On the other hand, the receiving device 3 includes a receiving unit 31, a signal processing unit 32, memory 33, an interface (I/F) unit 34, an operation unit 35, a display unit 36, and a battery 37. The receiving unit 31 receives, via the receiving antennas 41a to 41h, the in-vivo image data wirelessly transmitted from the capsule endoscope 2. The signal processing unit 32 controls each unit in the receiving device 3, and performs the predetermined signal processing on the in-vivo image data received by the receiving unit 31. The memory 33 stores various types of operations executed by the signal processing unit 32, the in-vivo image data having been subjected to signal processing by the signal processing unit 32, and related information related thereto (the received strength information, the time information, and the like). The interface unit 34 transmits the image data stored in the memory 33 to the image display apparatus 5 via the cradle 5a. The operation unit 35 is used by the user to input various types of operation instructions and settings to the receiving device 3. The display unit 36 notifies or displays various types of information to the user. The battery 37 supplies electric power to each unit in the receiving device 3.

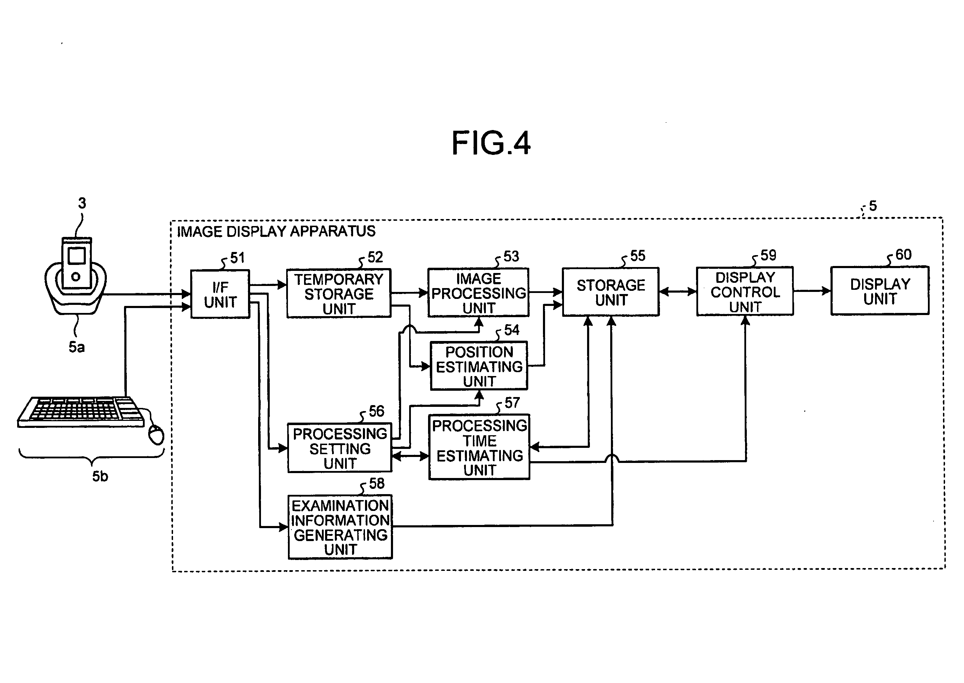

FIG. 4 is a block diagram illustrating the configuration of the image display apparatus 5.
As illustrated in FIG. 4, the image display apparatus 5 includes an interface (I/F) unit 51, a temporary storage unit 52, an image processing unit 53, a position estimating unit 54, a storage unit 55, a processing setting unit 56, a processing time estimating unit 57, an examination information generating unit 58, a display control unit 59, and a display unit 60. Among them, the image processing unit 53, the position estimating unit 54, the processing setting unit 56, the processing time estimating unit 57, the examination information generating unit 58, and the display control unit 59 are physically configured with a CPU (central processing unit).

The interface unit 51 functions as an input receiving unit for receiving the in-vivo image data and the related information related thereto, which are input via the cradle 5a, and receiving various types of instructions and information, which are input via the operation input device 5b.

The temporary storage unit 52 is configured with volatile memory such as DRAM and SDRAM, and temporarily stores the in-vivo image data which are input from the receiving device 3 via the interface unit 51. Alternatively, instead of the temporary storage unit 52, a recording medium and a drive device for driving the recording medium, such as an HDD (hard disk drive), an MO (magneto-optical disks), a CD-R, and a DVD-R, may be provided, and the in-vivo image data which are input via the interface unit 51 may be temporarily stored to the recording medium.

The image processing unit 53 performs on the in-vivo image data stored in the temporary storage unit 52, basic (essential) image processing such as white balance processing, demosaicing, color conversion, density conversion (such as gamma conversion), smoothing (noise reduction and the like), and sharpening (edge emphasis and the like), and auxiliary (optional) image processing for detecting lesion site and the like. Examples of optical image processing include red detecting processing for detecting a bleeding point and detection of lesion site due to tumor, blood vessel, tumor, and the like achieved by image recognition processing.

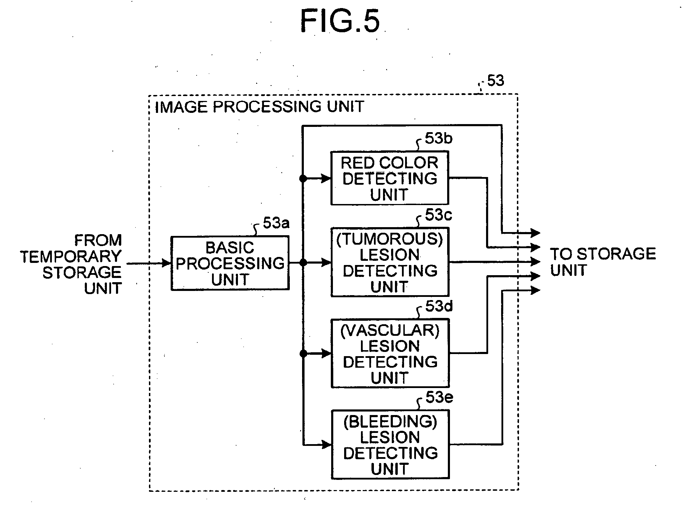

FIG. 5 is a figure illustrating the detailed operation of the image processing unit 53. The image processing unit 53 includes a basic processing unit 53a for performing basic image processing such as white balance processing and demosaicing on the in-vivo image data, a red color detecting unit 53b for performing the optional image processing, a (tumorous) lesion detecting unit 53c, a (vascular) lesion detecting unit 53d, and a (bleeding) lesion detecting unit 53e. First, the in-vivo image data stored in the temporary storage unit 52 are retrieved into the basic processing unit 53a, so that the basic image processing is performed on the in-vivo image data. The image-processed in-vivo image data are stored to the storage unit 55 and retrieved into a predetermined detecting unit among the red color detecting unit 53b to (bleeding) lesion detecting unit 53e, so that processing is performed in parallel. A detection result provided by the basic processing unit 53a is stored to the storage unit 55.

The position estimating unit 54 executes position estimating processing on the basis of the received strength information and the time information stored in the temporary storage unit 52. More specifically, the position estimating unit 54 obtains, from the temporary storage unit 52, the received strength of each of the receiving antennas 41a to 41h associated with the in-vivo image data received at a certain time, and extracts a spherical region of which center is at each of the receiving antennas 41a to 41h and of which radius is a distance according to the received strength. The weaker the received strength is, the larger the radius is. The position where these regions cross each other is estimated as a position of the capsule endoscope 2 at that time, i.e., the position represented by the in-vivo image in the subject 10. The position estimating unit 54 executes this kind of position estimation at a predetermined sampling density. The position thus estimated (estimated position information) is associated with the time information and stored to the storage unit 55.
It should be noted that various known methods other than the above can be applied as a specific method of this position estimating processing.

The storage unit 55 stores, e.g., not only parameters and various types of processing programs executed by the image display apparatus 5 but also the in-vivo image data subjected to the image processing by the image processing unit 53, the estimated position information obtained by the position estimating unit 54, and examination information generated by the examination information generating unit 58 explained later. For example, the storage unit 55 is configured with a recording medium and a drive device for driving the recording medium, such as a semiconductor memory such as flash memory, RAM (Random Access Memory), ROM (Read Only Memory), and an HDD (hard disk drive), an MO (magneto-optical disks), a CD-R, and a DVD-R.

The processing setting unit 56 sets the content of the image processing executed by the image processing unit 53 (the type and the precision of the image processing) and the position estimation level of the position estimating processing executed by the position estimating unit 54, on the basis of information that is input via the interface unit 51 when the user manipulates the operation input device 5b (input information), thus controlling the image processing unit 53 and the position estimating unit 54 so as to execute each processing with the content having been set (hereinafter referred to as processing content).

More specifically, the processing setting unit 56 sets, on the basis of the above input information, at least one detecting unit or estimating unit that executes processing, from among the red color detecting unit 53b, the (tumorous) lesion detecting unit 53c, the (vascular) lesion detecting unit 53d, the (bleeding) lesion detecting unit 53e, and the position estimating unit 54. Further, with regard to the position estimating processing, the processing setting unit 56 sets, on the basis of the input information, any one of, e.g., three position estimation levels (of which estimation precisions are low, medium, and high, respectively) that can be executed.

The processing time estimating unit 57 predicts a time needed in the image processing and/or position estimating processing (processing time) on the basis of the content set by the processing setting unit 56. It should be noted that the processing time in the first embodiment does not mean a simple summation of times required by the image processing and the position estimating processing but means an actual processing time from when the image processing and the position estimating processing are started to be executed in parallel to when the image processing and the position estimating processing are completed.

More specifically, the processing time estimating unit 57 predicts the processing time on the basis of the elements (1) to (4) as follows.

### (1) CPU occupation rate

The image display apparatus 5 executes, in parallel, not only the processing on the in-vivo image data but also various types of processing such as initialization of the memory of the receiving device 3 after the transfer, generation of the examination information, and the display of the existing in-vivo image. Accordingly, the CPU occupation rate changes from time to time. The processing time estimating unit 57 predicts and calculates, in accordance with the occupation rate at that time, the processing time required when the image processing and the position estimating processing are executed in parallel. It should be noted that, when multiple sets of image processing are executed, various types of image processing are basically executed in parallel, but under a circumstance where, for example, the CPU occupation rate is high, and the number of sets of processing that can be processed in parallel is limited, various types of image processing may be processed serially. In view of such situation, the processing time estimating unit 57 predicts the processing time.

### (2) Processing time per in-vivo image (or the amount of data processing per in-vivo image)

The storage unit 55 stores, as parameters, information about a time per in-vivo image (or the amount of data processing) that is required in various types of image processing including the red color detecting processing, the tumorous lesion detecting processing, the vascular lesion detecting processing, and the bleeding lesion detecting processing, and the position estimating processing. The processing time estimating unit 57 retrieves, from the storage unit 55, the information about the time required in the image processing and the position estimating processing which are set by the processing setting unit 56.

### (3) The number of in-vivo images

The amount of data processing in the image processing and the position estimating processing greatly changes in accordance with the amount of in-vivo image data transferred from the receiving device 3. In other words, the amount of data processing in the image processing and the position estimating processing greatly changes in accordance with the number of in-vivo images. The number of in-vivo images is determined in accordance with the image capturing rate of the capsule endoscope 2 and the examination time (a time from when the capsule endoscope 2 is introduced into the subject 10 to when the capsule endoscope 2 is excreted out of the subject 10).

### (4) Sampling density corresponding to precision of each position estimation

The storage unit 55 stores, as a parameter, sampling density information corresponding to each position estimation level (low, medium, and high). The processing time estimating unit 57 retrieves, from the storage unit 55, the sampling density information corresponding to the position estimation level set by the processing setting unit 56. The total number of in-vivo images to be subjected to the position estimating processing is determined in accordance with the sampling density and the number of in-vivo images.

Further, the processing time estimating unit 57 may obtain the finish time of the image processing and the position estimating processing, on the basis of the calculated processing time and the current time.

The examination information generating unit 58 generates information about the examination on the basis of the information provided via the operation input device 5b. More specifically, the information includes patient information (ID, name, sex, age, date of birth, and the like) for distinguishing the subject 10, who is a patient, and also includes diagnosis information for identifying the content of diagnosis of the subject 10 (the name of a hospital, the name of a doctor (nurse) who give the capsule, the date and time when the capsule was given, the date and time when the data were obtained, the serial number of the capsule endoscope 2, the serial number of the receiving device 3, and the like). It should be noted that the examination information may be generated in advance before the receiving device 3 transfers the in-vivo image data, or may be generated after the in-vivo image data are transferred.

The display control unit 59 controls the display unit 60 so as to display, in a predetermined format, the in-vivo image having been subjected to the image processing by the image processing unit 53, the position information estimated by the position estimating unit 54, and various types of other information.

The display unit 60 is configured with a CRT display or a liquid crystal display, and under the control of the display control unit 59, the display unit 60 displays various types of information and the radiographic image interpretation screen including the in-vivo image of the subject 10.

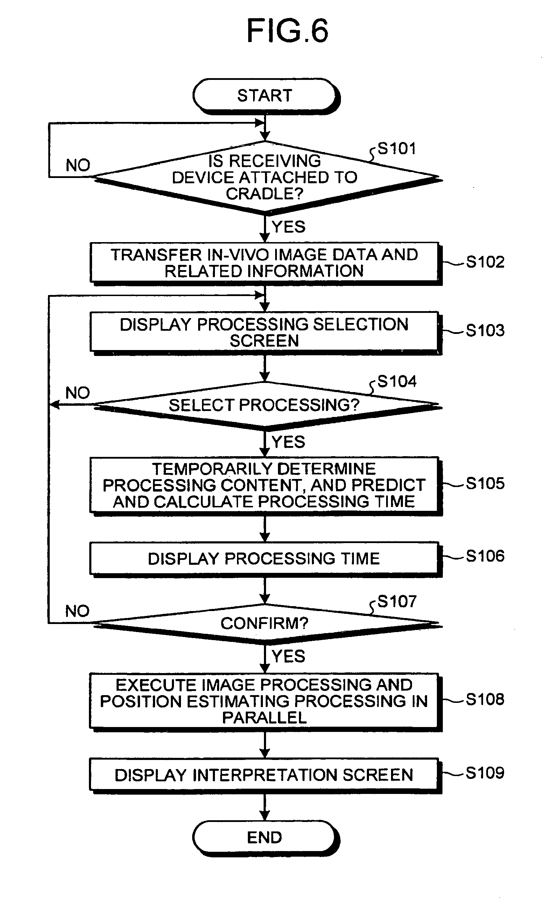

Subsequently, operation of the image display apparatus 5 will be explained with reference to FIG. 6. FIG. 6 is a flowchart illustrating operation of the image display apparatus 5.
When the receiving device 3 is attached to the cradle 5a in step S101 (step S101: Yes), the in-vivo image data and the related information related thereto, which are stored in the memory of the receiving device 3, are begun to be transferred to the image display apparatus 5 (step S102). At this occasion, the transferred in-vivo image data and the like are stored to the temporary storage unit 52. When the receiving device 3 is not attached to the cradle 5a (step S101: No), the image display apparatus 5 waits until the receiving device 3 is attached.

When the transfer of the in-vivo image data and the related information related thereto is finished, each unit of the image display apparatus 5 sets the processing content of the position estimating processing and the image processing in steps S103 to S107. First, in step S103, the display control unit 59 controls the display unit 60 for displaying the screen for allowing the user to select a desired processing content.

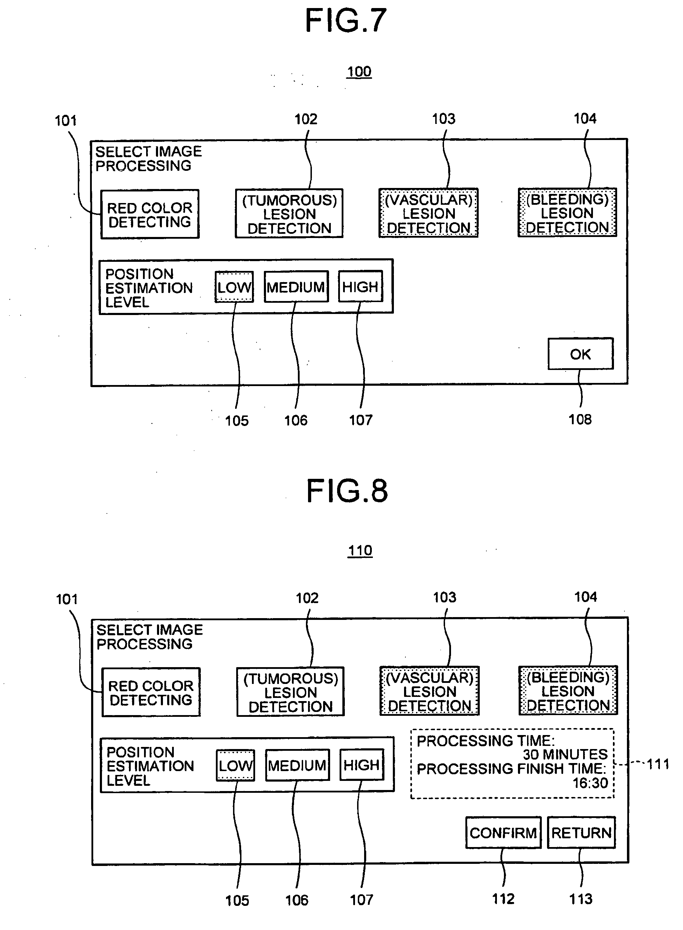

FIG. 7 is a schematic diagram illustrating an example of display of such selection screen. In a processing selection screen 100 as illustrated in FIG. 7, the user can select processing content performed on the in-vivo image data stored in the temporary storage unit 52 by performing pointer operation using the operation input device 5b such as a mouse. More specifically, the processing selection screen 100 includes icons 101 to 104 representing the type of any image processing (red color detecting, (tumorous) lesion detection, (vascular) lesion detection, (bleeding) lesion detection), icons 105 to 107 representing the position estimation level (low, medium, and high), and an OK button 108. Every time the icons 101 to 107 are clicked by pointer operation on the screen using a mouse and the like, the icons 101 to 107 are switched between the selected state and the unselected state.

When this processing selection screen 100 is initially displayed, all of the icons 101 to 104 are selected, and the icon 106 of the icons 105 to 107 is selected. In other words, the processing selection screen 100 indicates that all the image processing and the position estimation at the precision "medium" are executed. From this screen, the icon (for example, the icons 101 and 102) representing unnecessary image processing is unselected, and the icon (for example, icon 105) representing the desired position estimation level is selected. FIG. 7 illustrates a state after the processing content is changed as described above.

When the type of the image processing and the position estimation level desired by the user are selected and further a selection signal of the OK button 108 is input (for example, the OK button is clicked) in the processing selection screen 100 (step S104: Yes), the processing setting unit 56 primarily determines the processing content displayed on the processing selection screen 100, and the processing time estimating unit 57 predicts and calculates the processing time required to execute the determined processing content (step S105). When the selection signal of the OK button 108 is input (step S104: No), this processing selection screen 100 is continuously displayed on the display unit 60 (step S103), and the user can select the processing content again and again for any number of times.

Subsequently, in step S106, the display control unit 59 causes the display unit 60 to display the processing time calculated by the processing time estimating unit 57. FIG. 8 is a schematic diagram illustrating an example of display of the processing time. In FIG. 8, a processing time confirmation screen 110 includes not only the icons 101 to 107 but also a prediction processing time display field 111, a "confirm" button 112, and a "return" button 113. The prediction processing time display field 111 displays a processing time (estimated processing time) calculated by the processing time estimating unit 57 and the finish time of the processing (estimated finish time). The user sees this processing time confirmation screen 110 to confirm a waiting time until start of the interpretation of the images and a time at which the interpretation of the images can be started.

When a selection signal of the "confirm" button 112 is input on the processing time confirmation screen 110 (step S107: Yes), the processing setting unit 56 determines the selected processing content, and causes the image processing unit 53 and the position estimating unit 54 to execute the processing based on this content in parallel (step S108). On the other hand, when a selection signal of the "return" button 113 is input in the processing time confirmation screen 110 (step S107: No), the display control unit 59 displays the processing selection screen 100 as illustrated in FIG. 7 on the display unit 60 again (step S103). The user can select the processing content all over again in the processing selection screen 10.

In step S108, the position estimating unit 54 executes the position estimating processing at the position estimation level having been set, in parallel with the processing of the image processing unit 53.

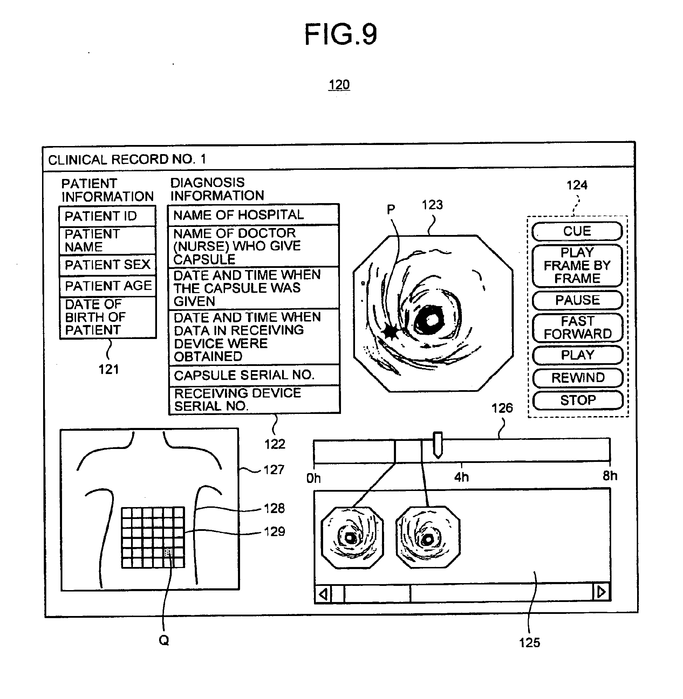

When the image processing and the position estimating processing as described above are finished, the display control unit 59 causes the display unit to display the radiographic image interpretation screen including the in-vivo image and the estimated position information in step S109. FIG. 9 is a schematic diagram illustrating an example of display of the radiographic image interpretation screen. In FIG. 9, a radiographic image interpretation screen 120 includes a patient information region 121 for displaying the identification information of the subject 10 who is the patient, a diagnosis information region 122 for displaying the identification information about the diagnosis of the subject 10, a main display region 123 in which a series of in-vivo images are reproduced, a reproduction operation button group 124 for performing reproduction operation of the in-vivo images displayed in the main display region 123, a thumbnail region 125 for displaying, as thumbnails, reduced images of multiple in-vivo images, a time bar 126 representing times at which the in-vivo images currently displayed in the main display region 123 were obtained, and a position display region 127. In the radiographic image interpretation screen 120, the reduced images in the thumbnail region 125 and points of the time bar 126 representing the times at which the reduced images were obtained are connected by lines. In the position display region 127, a person-shaped image 128 mimicking the subject 10 is displayed, and multiple regions (divided regions) 129 divided in a matrix form is displayed so as to be overlaid thereon.

In the main display region 123, the in-vivo image corresponding to the in-vivo image data processed by the basic processing unit 53a as illustrated in FIG. 5 is displayed, and a lesion site P detected by the red color detecting unit 53b to (bleeding) lesion detecting unit 53e is displayed so as to be overlaid thereon. In the position display region 127, a position Q estimated as a position where the in-vivo image currently displayed in the main display region 123 was taken is displayed. The user diagnoses lesion sites and the like while the user looks at the radiographic image interpretation screen.

As explained above, in the image display apparatus according to the first embodiment, the processing content desired by the user is executed on the in-vivo image data, so that the waiting time until the start of the interpretation of the images can be reduced to the minimum. In addition, the time required in the processing on the in-vivo image data or the finish time of the processing is predicted and displayed, and this enables the user to efficiently utilize the waiting time until the start of the interpretation of the images.

In the first embodiment, four types of image processing are mentioned. However, the types of image processing are not limited to the four types as described above. In other words, the number of the types of image processing may be increased or decreased to any number as long as it is possible to select whether to execute or not to execute at least one type of image processing.
In the first embodiment, a case where both of the position estimating processing and the image processing are executed has been explained as a specific example. Alternatively, at least one of the position estimating processing and the image processing may be executed.

### (Modification 1-1)

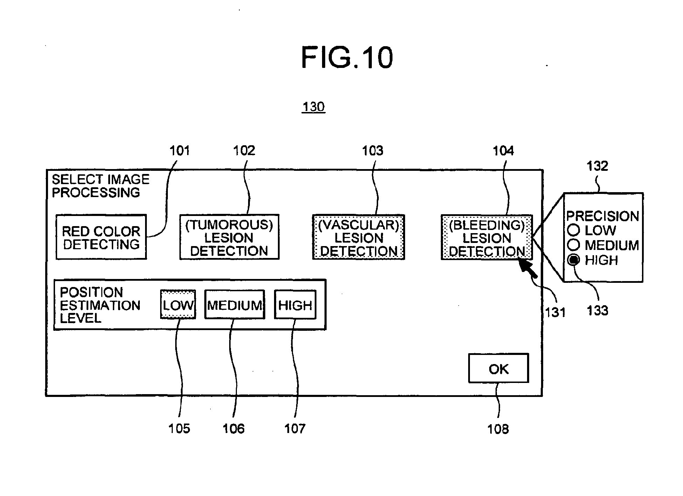

Subsequently, the first modification of the image display apparatus according to the first embodiment will be explained with reference to FIG. 10. In the first embodiment, the user is allowed to select the type of image processing executed optionally. Alternatively, the user may be allowed to select the precision of various types of image processing. FIG. 10 illustrates a processing selection screen displayed in the present modification. In this modification 1-1, the sampling density information corresponding to the precision of various types of image processing is stored to the storage unit 55 as a parameter in advance.

When a predetermined operation signal is input (for example, a cursor 131 is placed on any one of the icons 101 to 104, and the mouse is right-clicked) in a processing selection screen 130 as illustrated in FIG. 10, the display control unit 59 causes the display unit 60 to display a precision selection window 132 for selecting the precision of the image processing (for example, three levels, i.e., low, medium, and high). The precision selection window 132 includes radio buttons 133 corresponding to three levels of precision. It should be noted that only one of these radio buttons 133 can be selected at a time.

When the user clicks and selects the radio button 133 of any one of the levels of precision through pointer operation on the screen using a mouse and the like, the processing time estimating unit 57 retrieves the sampling density information corresponding to the selected precision from the storage unit 55, and predicts and calculates the processing time. After the processing content is determined, the processing setting unit 56 causes the red color detecting unit 53b to (bleeding) lesion detecting unit 53e of the image processing unit 53 to execute the image processing at the sampling density.

According to this modification 1-1, the user can efficiently interpret the in-vivo images which have been subjected to the desired image processing at the desired precision.

### (Modification 1-2)

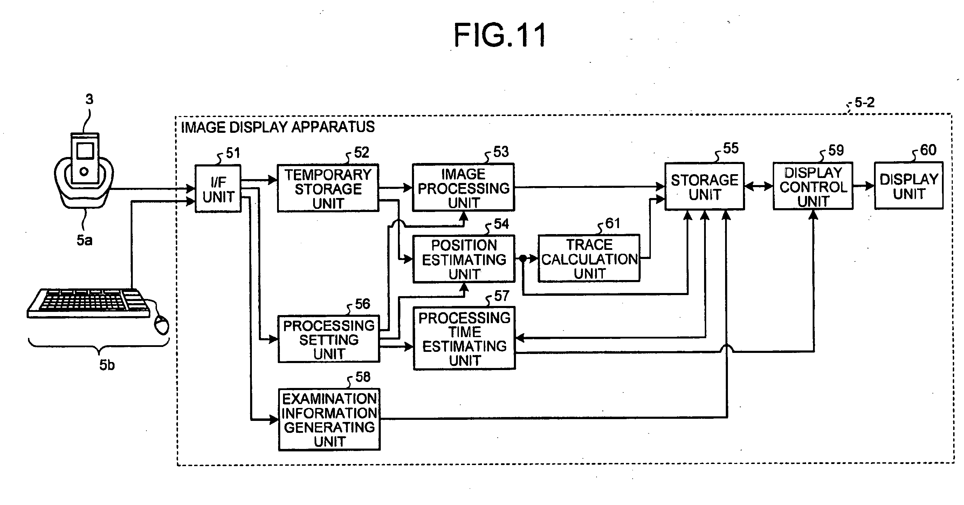

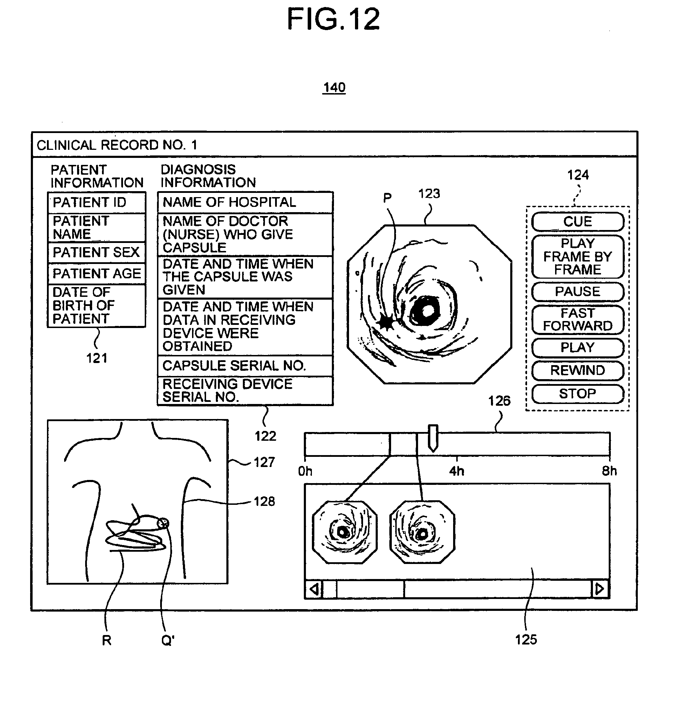

Subsequently, the second modification of the image display apparatus according to the first embodiment will be explained with reference to FIGS. 11 and 12. FIG. 11 is a block diagram illustrating a configuration of the image display apparatus according to the modification 1-2. In addition to FIG. 4, this image display apparatus 5-2 further includes a trace calculation unit 61 at a stage subsequent to the position estimating unit 54. The configuration other than the above is the same as the configuration of the image display apparatus 5.

The trace calculation unit 61 executes trace calculation processing of the capsule endoscope 2 on the basis of the estimated position information obtained by the position estimating unit 54. More specifically, the trace calculation unit 61 extracts, from multiple estimated positions of the capsule endoscope 2, two points adjacent to each other in terms of time, and when the distance between these two points is equal to or less than a predetermined value, the two points are connected. By doing so, the trace calculation unit 61 successively connects the estimated positions, thus calculating the total trace and generating trace information. It should be noted that this trace information is stored to the storage unit 55. It should be noted that various known methods other than the above can be applied as a specific method of this trace calculation processing.

For example, the display control unit 59 displays a trace on a radiographic image interpretation screen 140 as illustrated in FIG. 12 on the basis of the trace information thus generated. More specifically, instead of the divided region 129 (see FIG. 9) on the person-shaped image 128 of the position display region 127, a trace R is drawn. Further, the display control unit 59 may display, on the trace R in an overlapping manner, a position Q' of the in-vivo image currently displayed in the main display region 123.

If the image processing by the image processing unit 53 and the position estimating processing by the position estimating unit 54 are finished, the display control unit 59 may start displaying the radiographic image interpretation screen even before the trace calculation processing is finished. In this case, first, in the radiographic image interpretation screen 120 as illustrated in FIG. 9, simplified position indication may be illustrated on the basis of the estimated position information, and when the trace calculation processing is finished, the trace R based on the trace information may begun to be displayed in the radiographic image interpretation screen 140 as illustrated in FIG. 12.

According to this modification 1-2, the user can see the trace to more correctly understand the position of the in-vivo image in question.

### Second embodiment

Subsequently, an image display apparatus according to a second embodiment of the present invention will be explained. The configuration of the image display apparatus according to the second embodiment is the same as the one as illustrated in FIG. 4, and is different from the first embodiment in operation for setting the processing content in an image processing unit 53 and/or a position estimating unit 54.

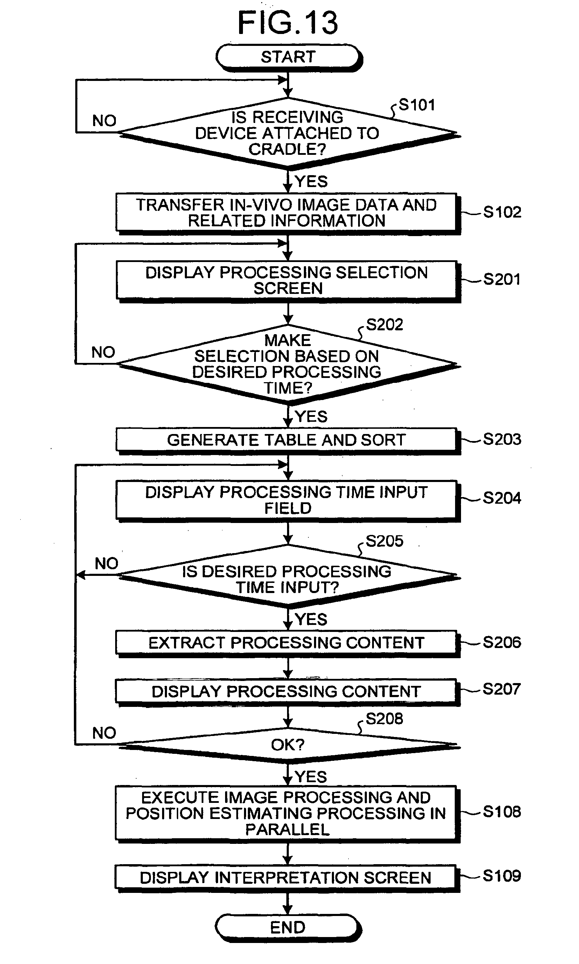

FIG. 13 is a flowchart illustrating processing content setting operation executed by the image display apparatus according to the second embodiment.
First, in steps S101 and S102, in-vivo image data and related information related thereto are transferred from a receiving device 3 attached to a cradle 5a to an image display apparatus 5. The details of these steps are the same as those explained in the first embodiment (see FIG. 6).

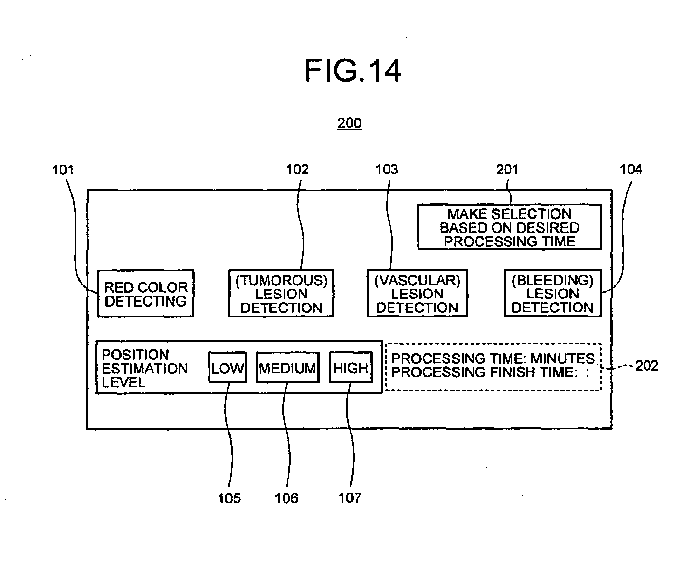

In step S201, the display control unit 59 causes the display unit 60 to display a screen for allowing a user to select whether to enter into a mode for selecting a processing content in a desired processing time. FIG. 14 is a schematic diagram illustrating an example of display of such screen. Like FIG. 7, a processing selection screen 200 in FIG. 14 includes icons 101 to 104 representing the type of any image processing and icons 105 to 107 representing the position estimation level, and further includes a selection button 201 for setting the processing content of the image processing and/or the position estimating processing using a processing time desired by the user as a condition. The processing selection screen 200 is provided with a predicted time display region unit 202 for displaying an estimated processing time taken to execute the image processing and/or the position estimating processing with the processing content thus set and a predicted processing finish time.

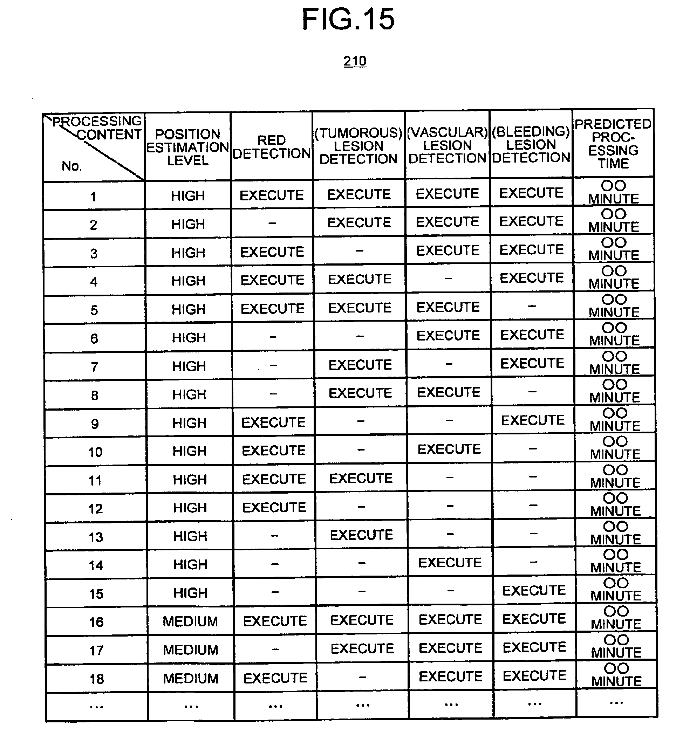

When a selection signal of the selection button 201 is input in this processing selection screen 200 (step S202: Yes), for example, the processing setting unit 56 generates a processing content table 210 as illustrated in FIG. 15 (step S203). This processing content table 210 includes a processing content including combinations of the type of image processing and the position estimation level, serving as candidates of processing executed by the image processing unit 53 and the position estimating unit 54, and also includes a prediction processing time taken to execute each processing content. Among them, the prediction processing time is calculated by the processing time estimating unit 57 on the basis of the CPU occupation rate and the number of in-vivo images at that time. Further, the processing setting unit 56 sorts the processing contents in accordance with the calculated prediction processing times.

On the other hand, when a selection signal of the selection button 201 is not input (step S202: No), the display control unit 59 repeats display of the processing selection screen 200 as illustrated in FIG. 14 (step S201). When the selection signal of the selection button 201 is not input, the user may directly select the icons 101 to 107 to set a processing content like the first embodiment.

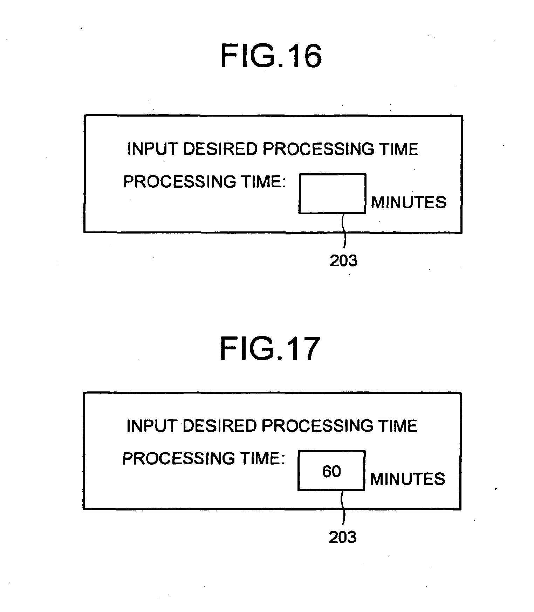

In step S204, for example, the display control unit 59 causes the display unit 60 to display a processing time input field 203 as illustrated in FIG. 16.
As illustrated in FIG. 17, when a desired processing time (for example, 60 minutes) is input to the processing time input field 203 (step S205: Yes), the processing setting unit 56 extracts, from the processing content table 210, a processing content of which prediction processing time is closest to the desired processing time (step S206). At this occasion, when there are multiple applicable processing contents (for example, a case where there are a processing content of which prediction processing time is the desired processing time + α and a processing content of which prediction processing time is the desired processing time - α), the processing content of which prediction processing time is within the desired processing time (the processing content of which prediction processing time is the desired processing time - α) may be preferentially extracted.
When the desired processing time is not input to the processing time input field 203 (step S205: No), the processing time input field 203 is continued to be displayed (step S204).

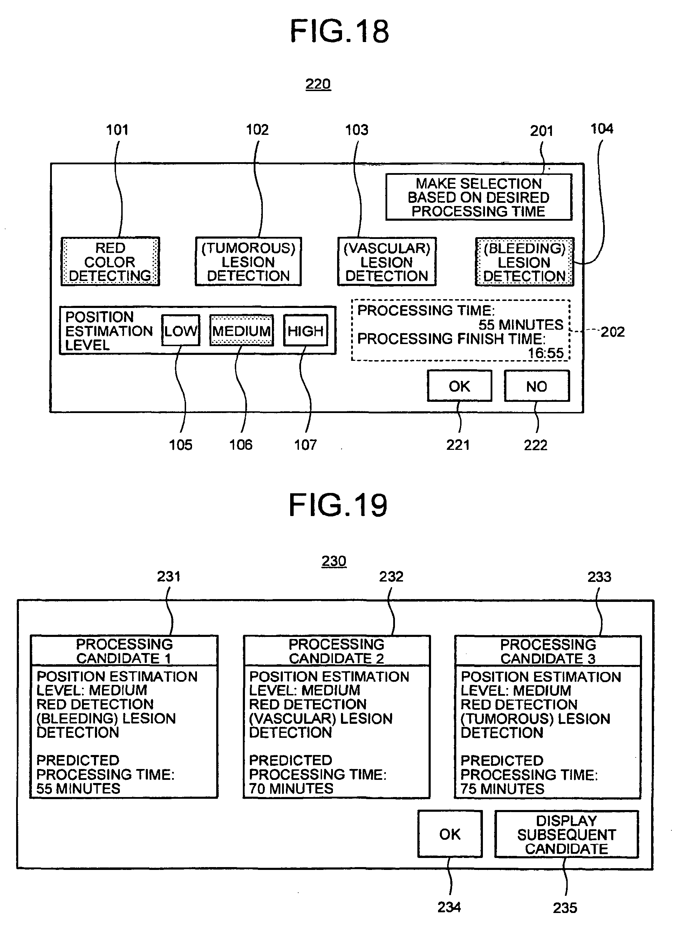

In step S207, the display control unit 59 causes the display unit 60 to display the extracted processing content. FIG. 18 is a schematic diagram illustrating an example of display of processing content. In a processing display screen 220 as illustrated in FIG. 18, icons representing the extracted processing content is displayed, e.g., in a highlighted manner or blinked. It should be noted that the processing display screen 220 shows a state in which a red color detecting processing (icon 101), a bleeding lesion detecting processing (icon 104), and a position estimation level "medium" (icon 106) are selected. The predicted time display region unit 202 displays a prediction processing time "55 minutes" and a processing finish time "16:55" for a case where the above processing is executed.

When a selection signal of an OK button 221 is input in the processing display screen 220 (step S208: Yes), the processing setting unit 56 determines the displayed processing content. Thereafter, the image processing and the position estimating processing are executed in parallel (step S108), and the radiographic image interpretation screen is displayed on the display unit 60 (step S109). It should be noted that operation in steps S108 and S109 is the same as the operation explained in the first embodiment (see FIG. 6).

On the other hand, when a selection signal of a NO button 222 is input in the processing display screen 220 (step S208: No), the display control unit 59 causes the display unit 60 to display the processing time input field 203 as illustrated in FIG. 16 again (step S204). Therefore, the user can input all over again from the input of the processing time. Alternatively, when the selection signal of the NO button 222 is input (step S208: No), the user may be allowed to select whether to enter into the mode for selecting a processing content according to the desired processing time (step S201, see FIG. 14). When the mode is thereafter selected again (step S202: Yes), step S203 is preferably skipped because the processing content table (see FIG. 15) has already been generated.

As described above, according to the second embodiment, the processing content is selected according to the desired processing time, and the user can efficiently interpret the radiographic images on the basis of the in-vivo images having been subjected to necessary processing in a limited time.

It should be noted that each processing content illustrated in the processing content table 210 of FIG. 15 includes both of the position estimating processing and any one of image processing, but in the second embodiment, at least one of the position estimating processing and various types of image processing may be included. In other words, a processing content including only the position estimating processing or one or more types of image processing may be set.

In step S205 explained above, instead of having the user input the desired processing time, a desired finish time may be input. In this case, the processing time estimating unit 57 calculates the desired processing time based on the desired finish time that has been input, and extracts a processing content in accordance with the desired processing time.

When the processing content displayed in step S206 explained above is not the processing content desired by the user, the user may be allowed to correct the processing content by selecting the icons 101 to 107 on the processing display screen 220. In this case, the processing time estimating unit 57 calculates the prediction processing time and the processing finish time again on the basis of the processing content, and the display control unit 59 controls the display unit 60 to display the result of recalculation in the predicted time display region unit.

### (Modification 2-1)

Subsequently, the first modification of the image display apparatus according to the second embodiment will be explained with reference to FIG. 19. In the second embodiment, the processing content of which prediction processing time is closest to the desired processing time is extracted. Alternatively, multiple processing contents may be displayed on the screen as candidates, and the user may be allowed to select them.

More specifically, when a desired processing time is input to a processing time input field 203 as illustrated in FIG. 17, the processing setting unit 56 extracts, from the processing content table 210 sorted according to the prediction processing time, processing contents (processing candidates 1 to 3) in such order that a processing content of which prediction processing time is closest to the desired processing time is extracted first. At this occasion, when there are multiple processing contents of which difference between the prediction processing time and the desired processing time is the same (for example, a case where there are a processing content of which prediction processing time is the desired processing time + α and a processing content of which prediction processing time is the desired processing time - α), these processing contents may be treated with the same level in the order, or the processing content of which prediction processing time is within the desired processing time (the processing content of which prediction processing time is the desired processing time - α) may be treated with a higher level in the order. In addition, the display control unit 59 causes the display unit 60 to display a processing candidate display screen 230 as illustrated in FIG. 19. The processing candidate display screen 230 includes icons 231 to 233 representing processing candidates 1 to 3, an OK button 234, and an instruction button 235 for displaying a subsequent candidate. In each of the icons 231 to 233, the type of any image processing and a position estimation level are displayed, and a processing time (prediction processing time) predicted when the processing is executed is also displayed.

When a selection signal of any one of the icons 231 to 233 and a selection signal of the OK button 234 are input, the processing setting unit 56 determines the processing content displayed in the selected icons 231 to 233, and causes the image processing unit 53 and the position estimating unit 54 to execute the processing.

On the other hand, when a selection signal of the instruction button 235 is input, the processing setting unit 56 extracts a subsequent processing candidate from the processing content table 210. Thereafter, the display control unit 59 displays, on the processing candidate display screen 230, an icon representing the subsequent processing candidate extracted by the processing setting unit 56. The user may select a desired processing content by way of an icon displayed on the display unit 60.

According to the modification 2-1, the user can compare multiple processing candidates, and therefore, the user can select a processing content that is more suitable for the user's request.

### (Modification 2-2)

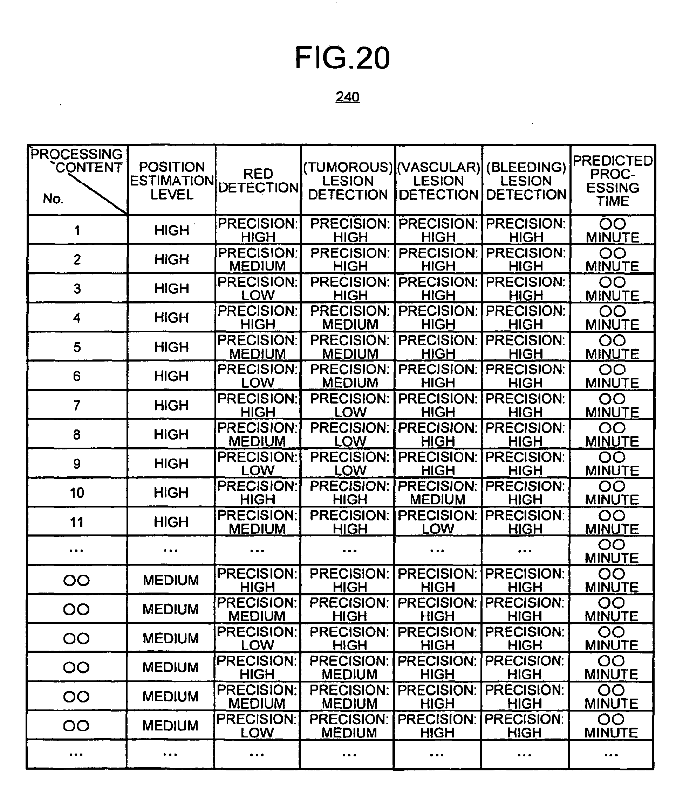

Subsequently, the second modification of the image display apparatus according to the second embodiment will be explained with reference to FIG. 20. In the modification 2-1 explained above, a combination of the type of image processing and the position estimation level is presented as a candidate. In addition, a precision of image processing may also be combined therewith.

More specifically, in step S203 as illustrated in FIG. 13, the processing setting unit 56 generates a processing content table 240 as illustrated in FIG. 20, for example. The processing content table 240 includes a processing content including a combination of a position estimation level, the type of image processing, and a precision (for example, three levels, i.e., low, medium, and high) in various types of image processing, and also includes a prediction processing time when each processing content is executed. The processing setting unit 56 extracts processing contents from the processing content table 240 in such order that a processing content of which prediction processing time is closest to the desired processing time that is input to the processing time input field 203 is extracted first. The display control unit 59 causes the display unit 60 to display icons representing the extracted processing contents, like the processing candidate display screen 230 as illustrated in FIG. 19. The user may select a desired processing content by way of an icon displayed on the display unit 60.
According to the modification 2-2, the precision of the image processing can also be selected, and therefore, the user can interpret radiographic in-vivo images having been subjected to the processing that is more suitable for the user's request.

### (Modification 2-3)

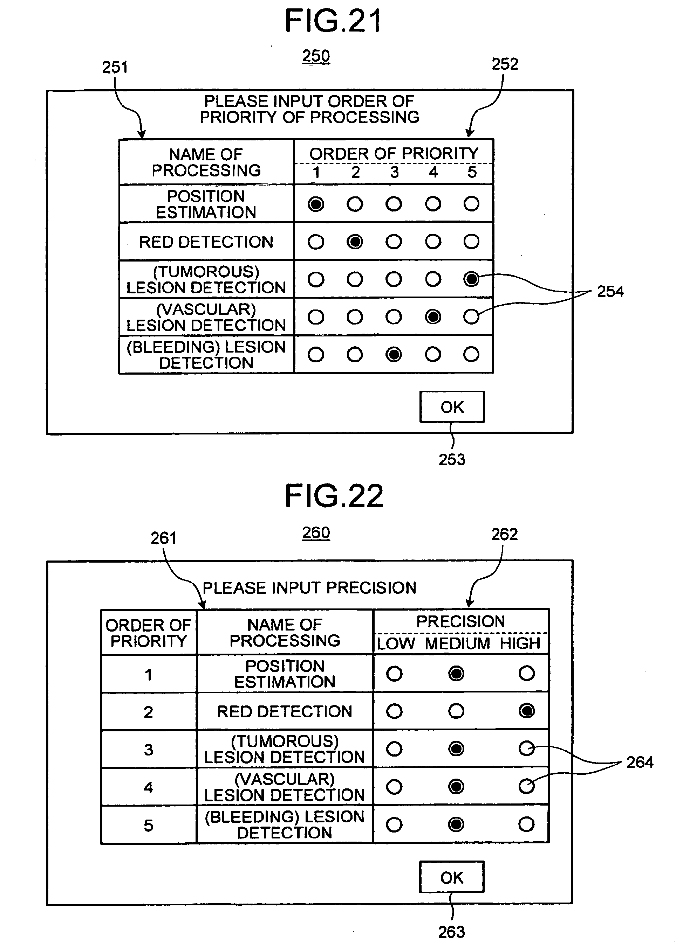

Subsequently, a third modification of the image display apparatus according to the second embodiment will be explained with reference to FIGS. 21 and 22. In the modification 2-2 explained above, the processing content is extracted only on the basis of the desired processing time. Alternatively, the type of image processing and the like may be set with the order of priority desired by the user in advance, and a processing content may be extracted on the basis of this order of priority and the desired processing time. It should be noted that the order of priority may be set at any time other than a time when the in-vivo image data are transferred.

When the order of priority is set, first, the display control unit 59 causes the display unit 60 to display a priority order setting screen 250 as illustrated in FIG. 21, for example. The priority order setting screen 250 includes a processing name field 251 describing processing name of the position estimating processing and various kinds of image processing, a priority order input field 252 into which the order of priority of each processing is input, and an OK button 253. The priority order input field 252 is constituted by a radio button 254 corresponding to the order, for example, as illustrated in FIG. 21. In the priority order input field 252, the order of priority selected by certain processing cannot be selected by other processing. The order of priority may not be selected for the image processing which is not required to be executed. It should be noted that FIG. 21 illustrates a case where the order of priority is given in the following order: the red color detecting processing, the (bleeding) lesion detecting processing, the (vascular) lesion detecting processing, and then the (tumorous) lesion detecting processing.

When the user selects a radio button 254 representing the order of priority desired by the user by pointer operation on the screen with a mouse and the like, and further the user selects the OK button 253, the display control unit 59 subsequently causes the display unit 60 to display a precision setting screen 260 as illustrated in FIG. 22. The precision setting screen 260 includes a processing name field 261 showing the position estimating processing and various kinds of image processing in the order set by the priority order setting screen 250, a precision input field 262 for selecting the precision of each processing, and an OK button 263. As illustrated in FIG. 22, for example, the precision input field 262 is constituted by radio buttons 264 corresponding to three levels of precision.

When the user selects a radio button 264 representing a desired precision of each processing, and further clicks the OK button 263, the processing setting unit 56 generates user setting information based on the content displayed on the precision setting screen 260, and stores this to a storage unit 55. It should be noted that FIG. 22 illustrates a case where the position estimation level is set at "medium", the processing precision of the red color detecting is at "high", each processing precision of the (tumorous) lesion detection, the (vascular) lesion detection, and the (bleeding) lesion detection is set at "medium".

When a desired processing time (for example, 60 minutes) is input to the processing time input field 203 as illustrated in FIG. 17 after the in-vivo image data are transferred, the processing time estimating unit 57 reads the user setting information stored in the storage unit 55, and, first, calculates a prediction processing time with the processing content of the user's setting. When this prediction processing time is within the desired processing calculation time, the display control unit 59 causes the display unit 60 to display the processing content of the user's setting in a format such as the processing display screen 220 as illustrated in FIG. 18, for example.

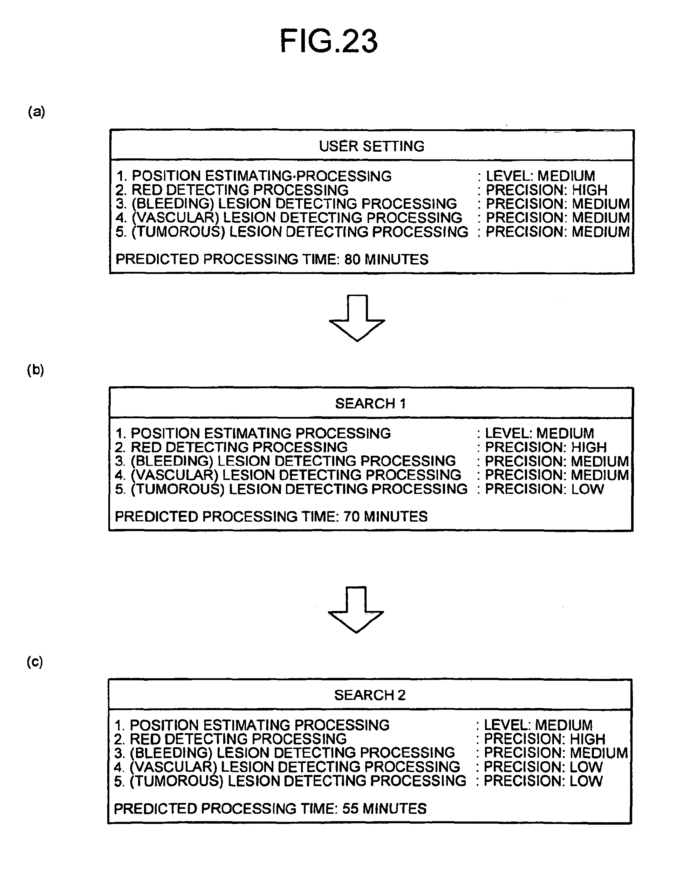

On the other hand, when the processing time is more than the desired processing time in the processing content of the user's setting prediction, the processing time estimating unit 57 searches a processing content which is close to the processing content of the user's setting as much as possible and of which prediction processing time fits within the desired processing time. FIG. 23 is a figure for explaining this search method.

For example, when the desired processing time is 60 minutes, as illustrated in FIG. 23(a), the prediction processing time (for example, 80 minutes) according to the processing content of the user's setting is 20 minutes longer than the desired processing time. Accordingly, the processing time estimating unit 57 reduces the prediction processing time by decreasing the precision of the processing (or the position estimation level) in the ascending order of priority. More specifically, as illustrated in FIG. 23(b), the first search (search 1) is performed such that the processing precision of (tumorous) lesion detection of which order of priority is the fifth is changed from "medium" to "low". Accordingly, the prediction processing time is reduced (for example, 70 minutes). Nevertheless, when the prediction processing time is more than the desired processing time, as illustrated in FIG. 23(c), the second search (search 2) is performed such that the processing time estimating unit 57 changes, from "medium" to "low", the processing precision of the (vascular) lesion detection of which order of priority is the fourth. Accordingly, the prediction processing time is further reduced (for example, 55 minutes), the prediction processing time fits within the desired processing time. For example, the display control unit 59 causes the display unit 60 to display the processing content obtained from the above search in a format such as the processing display screen 220 as illustrated in FIG. 18.

According to the modification 2-3, the order of priority unique to the user is set in advance, and therefore, the processing reflecting the user's preference can be performed on the in-vivo image data within the desired processing time.

### (Modification 2-4)

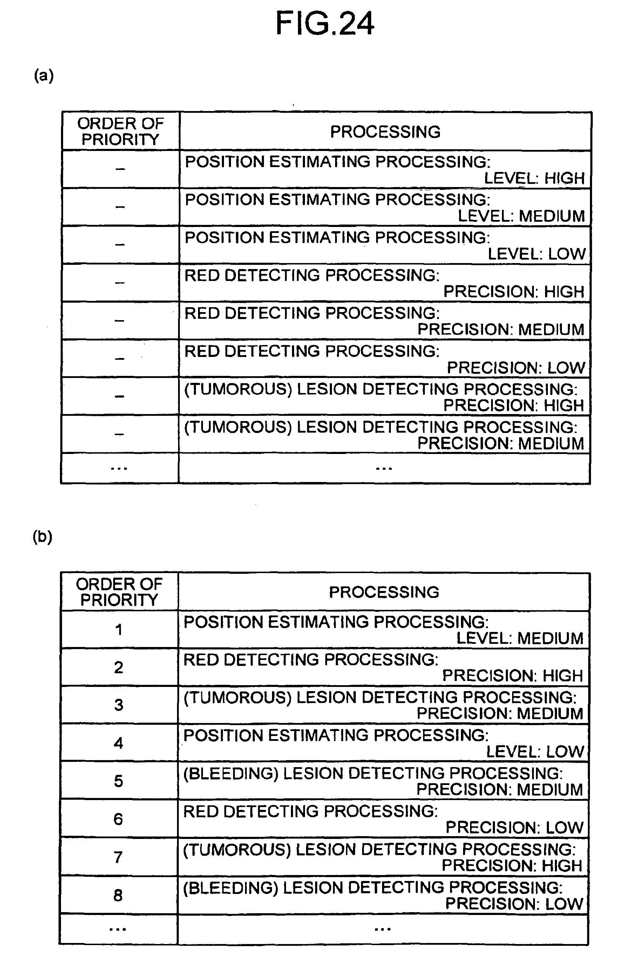

Subsequently, the fourth modification of the image display apparatus according to the second embodiment will be explained with reference to FIG. 24. In the present modification, as illustrated in FIG. 24, the order of priority is also set for the position estimation level and the precision of various kinds of image processing. FIG. 24(a) illustrates a table enumerating the precision of various kinds of image processing and respective position estimation levels, and FIG. 24(b) illustrates a state in which the table as illustrated in FIG. 24(a) is sorted according to the order of priority of the user. The processing setting unit 56 generates the table having the order of priority thus set, and stores the table to the storage unit 55.

During the processing time, the processing setting unit 56 reads the table from the storage unit 55, and temporarily sets, as the processing content, processing of which order of priority is the highest (for example, position estimating processing "medium"). The processing time estimating unit 57 calculates the prediction processing time of the processing content temporarily set. When the prediction processing time is less than the desired processing time, the processing setting unit 56 adds processing of which order of priority is the second highest (for example, red color detecting processing "high"), thus setting a new processing content. The processing time estimating unit 57 calculates a prediction processing time of the newly set processing content (i.e., the position estimating processing "medium" and the red color detecting processing "high"). When the prediction processing time is less than the desired processing time, the processing setting unit 56 further adds processing of which order of priority is the subsequently higher (for example, (tumorous) lesion detecting processing "medium"), thus setting a new processing content. As described above, addition of the processing and calculation of the prediction processing time are repeated immediately before the prediction processing time becomes more than the desired processing time. The processing setting unit 56 determines that the processing content immediately before the prediction processing time becomes more than the desired processing time is an ultimate processing content.

According to the modification 2-4, the order of priority is set in advance for the precision of the image processing, and the processing reflecting the user's preference can be performed on the in-vivo image data within the desired processing time.

Furthermore, like the modification 1-2, another modification of the second embodiment may be made by adding a trace calculation unit 61 to the second embodiment and the modifications 2-1 to 2-4 thereof.

### (Third embodiment)

Subsequently, an image display apparatus according to the third embodiment of the present invention will be explained. The configuration of the image display apparatus according to the third embodiment is the same as that as illustrated in FIG. 4. The flowchart illustrating operation of the image display apparatus according to the third embodiment is the same as that as illustrated in FIG. 6. In the present embodiment, operation for setting the processing content in the image processing unit 53 and/or the position estimating unit 54 is different from that of the first embodiment.

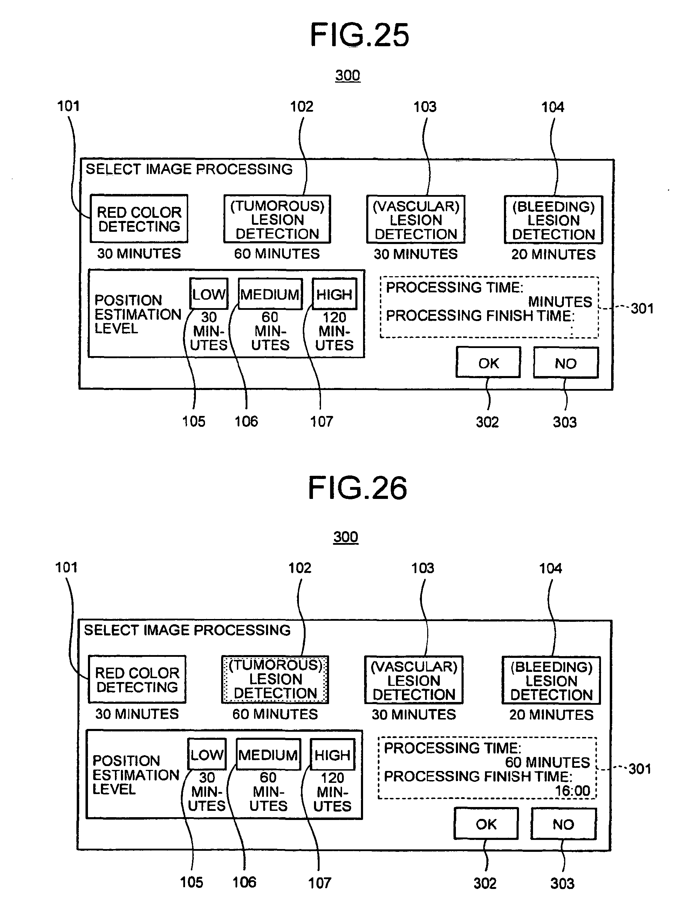

FIG. 25 illustrates an example of a processing selection screen displayed on a display unit 60 after in-vivo image data are transferred from the receiving device 3 to the image display apparatus 5. Like FIG. 7, a processing selection screen 300 includes icons 101 to 104 representing the types of image processing and icons 105 to 107 representing position estimation levels, and further includes a prediction time display region unit 301, an OK button 302, and a NO button 303. In the prediction time display region unit 301, a prediction processing time required in image processing and position estimating processing of the processing content having been set and a predicted processing finish time are displayed. Below the icons 101 to 107, individual processing times predicted where each processing is executed alone are displayed. In other words, in the third embodiment, the individual processing times displayed in the icons 101 to 107 are looked up, and a desired processing content fitting within the desired processing time can be selected by the user himself/herself.

When the user selects an icon representing a desired image processing and an icon representing a desired position estimation level by pointer operation on the processing selection screen 300 using a mouse and the like, the processing time estimating unit 57 calculates the prediction processing time required to execute the selected processing. Accordingly, the display control unit 59 causes the prediction processing time to display the calculated prediction processing time.

FIG. 26 illustrates a state in which only the icon 102 representing the (tumorous) lesion detecting processing is selected. In this case, the prediction processing time is 60 minutes which is the same as the individual processing time.

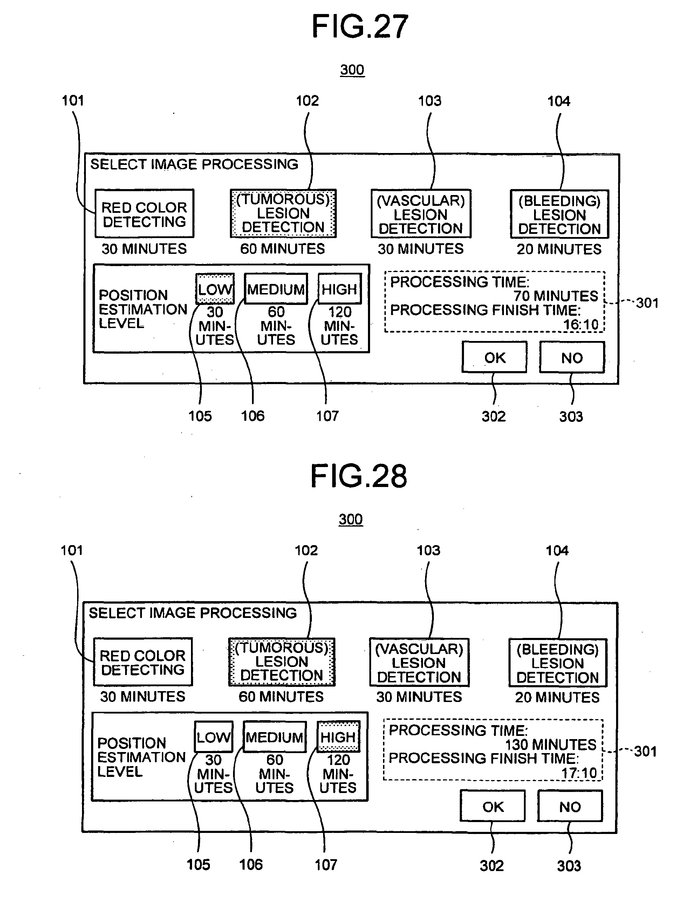

FIG. 27 illustrates a state in which not only the icon 102 but also the icon 105 representing a position estimation level "low" are selected. In this case, the position estimating processing and various kinds of image processing are executed in parallel, and the prediction processing time is not simply a summation of the individual processing time of the position estimating processing (30 minutes) and the individual processing time of the (tumorous) lesion detecting processing (60 minutes), but is an actual value (for example, 70 minutes).

FIG. 28 illustrates a state in which not only the icon 102 but also the icon 107 representing a position estimation level "high" are selected. In this case, for example, it takes 120 minutes to perform only the position estimating processing, and therefore, when the (tumorous) lesion detecting processing is executed in parallel, the prediction processing time becomes slightly longer than that (for example, 130 minutes).

When a selection signal of the OK button 302 is input in the processing selection screen 300 explained above, the processing setting unit 56 determines the selected processing content. On the other hand, when a selection signal of the NO button 303 is input, all the icons 101 to 107 are unselected. In this case, the user can select the icons 101 to 107 all over again from the beginning.

As described above, in the third embodiment, the individual processing time required to perform the position estimating processing and various kinds of image processing is displayed on the processing selection screen, and therefore, the user can look up the individual processing times to select a desired processing content. Therefore, the user himself/herself can adjust the desired processing content performed on the in-vivo image data and the time when the interpretation of the image can be started.

### (Modification 3)

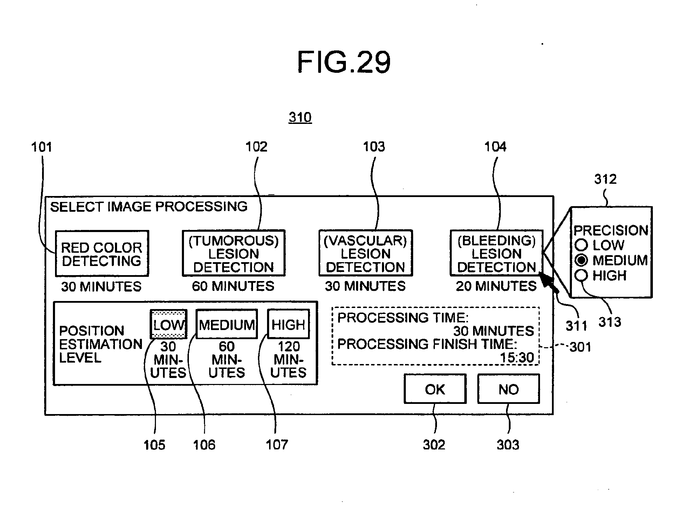

Subsequently, the modification of the image display apparatus according to the third embodiment will be explained with reference to FIG. 29. Like the modification 1-1 as illustrated in FIG. 10, the modification 3 may be configured such that the precisions of various kinds of image processing may be selected. In this case, a storage unit 55 previously stores sampling density information corresponding to the precisions of various kinds of image processing as parameters.

FIG. 29 illustrates an example of a processing selection screen displayed on a display unit 60 after in-vivo image data have been transferred from a receiving device 3 to an image display apparatus 5. Like FIGS. 25 to 28, a processing selection screen 310 includes icons 101 to 107, a prediction time display region unit 301, an OK button 302, and a NO button 303.

When a predetermined operation signal is input (for example, a cursor 331 is placed on any one of the icons 101 to 104, and it is right-clicked) in the processing selection screen 310, the display control unit 59 causes the display unit 60 to display a precision selection window 312 for selecting the precision of the image processing (for example, three levels, i.e., low, medium, and high). For example, the precision selection window 312 includes radio buttons 313 corresponding to three levels of precision. When the user clicks and selects a radio button 313 of any one of the levels of precision, the processing time estimating unit 57 retrieves the sampling density information corresponding to the selected precision from the storage unit 55, and calculates the individual processing time predicted where the image processing is executed independently. The display control unit 59 controls the display unit 60 to display the calculated individual processing times below the selected icon. For example, the individual processing time "20 minutes" required when the processing precision of the lesion detecting processing (bleeding) is "medium" is displayed below the icon 104 of FIG. 29. The user may look up each individual processing time thus displayed to select the desired processing content and the precision thereof.

Furthermore, like the modification 1-2, another modification of the third embodiment may be made by adding a trace calculation unit 61.

### (Fourth embodiment)

Subsequently, an image display apparatus according to the fourth embodiment of the present invention will be explained. The image display apparatus according to the fourth embodiment is based on the image display apparatus according to the third embodiment but is configured to allow selection of a portion in the subject 10 which is to be subjected to processing of in-vivo image data.

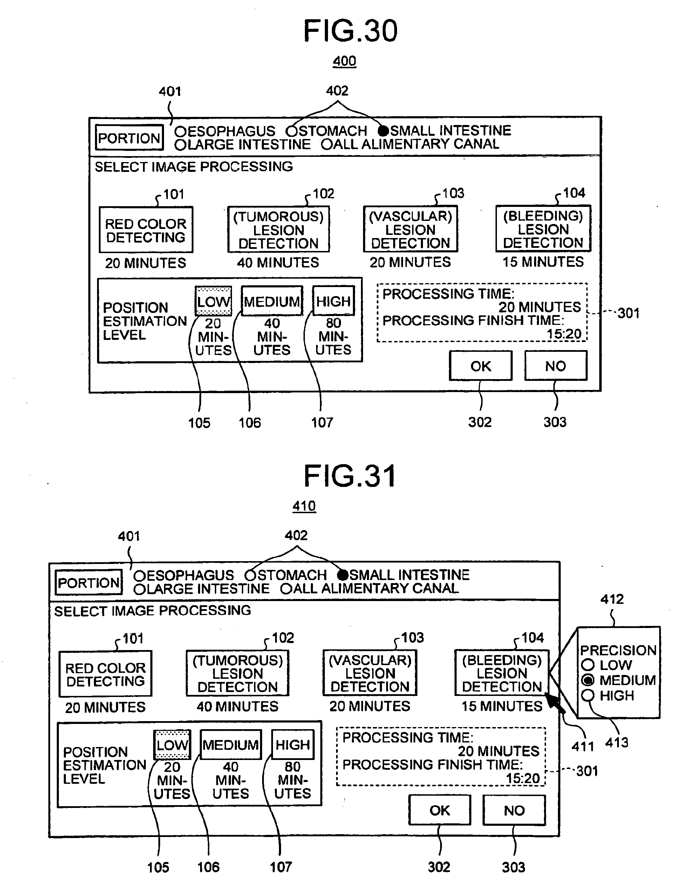

A processing selection screen 400 as illustrated in FIG. 30 additionally includes a portion selection field 401 in the processing selection screen 300 as illustrated in FIG. 25 to FIG. 28. The portion selection field 401 includes indications of "esophagus", "stomach", "small intestine", "large intestine", and "all alimentary canal", which are organs to be examined, and radio buttons 402 provided to correspond to the respective indications.

When the user selects any one of the radio buttons 402 in the processing selection screen 400, the processing setting unit 56 sets in-vivo image data in a range corresponding to a selected organ, as a target of processing of various kinds of image processing. More specifically, for example, it is assumed that in-vivo image data obtained within one hour from when the capsule endoscope 2 was introduced into the subject 10 are associated with the "esophagus", in-vivo image data obtained 30 minutes to 2 hours and half thereafter are associated with the "stomach", in-vivo image data obtained two hours to six hours and half thereafter are associated with the "small intestine", and in-vivo image data obtained six hours or more thereafter are associated with the "large intestine".

The processing time estimating unit 57 calculates the processing times of various kinds of image processing on the basis of the number of in-vivo images set as the target of processing. The display control unit 59 controls the display unit 60 so as to display the calculated processing times below the respective icons 101 to 104. It should be noted that FIG. 30 illustrates a case where the "small intestine" is selected as the target of processing.

According to the fourth embodiment, the user can look up the time of each processing displayed on the screen to select a desired processing content performed on a desired organ and a precision thereof. Therefore, the interpretation of the in-vivo images having been subjected to the desired processing can be started within a desired time.

It should be noted that the setting of the range of the in-vivo image data corresponding to the selected organ is not limited to the above explanation, and, for example, the range may be set on the basis of a mark given during examination with the capsule endoscope 2. More specifically, while the capsule endoscope 2 moves within the subject 10, the in-vivo image is displayed on the display unit 36 of the receiving device 3 as illustrated in FIG. 3 or in a viewer separately provided on the receiving device 3. The user observes the in-vivo image, and when the capsule endoscope 2 passes the border at each portion such as the stomach, the small intestine, and the large intestine, a mark is given to the in-vivo image. This marking information is transferred to the image display apparatus 5 as related information of the in-vivo image data, and based on the marking information, the portions represented by the in-vivo images may be classified.

### (Modification 4)

Subsequently, a modification of the image display apparatus according to the fourth embodiment will be explained with reference to FIG. 31. Like the modification 3 of the third embodiment explained above, the modification 4 may also be configured so as to allow selection of precisions of various kinds of images.

A processing selection screen 410 as illustrated in FIG. 31 is based on the processing selection screen 300 as illustrated in FIG. 30 but is configured to display a precision selection window 412 when a predetermined operation signal is input (for example, a cursor 411 is placed on any one of the icons 101 to 104, and it is right-clicked). For example, the precision selection window 412 includes radio buttons 413 corresponding to three levels of precision. When the user selects a desired precision of a desired image processing in the processing selection screen 410, a processing time required to execute the image processing with the selected precision is displayed below an icon representing the image processing (for example, the icon 104). The user may look up the time of each processing thus displayed to select the desired processing content for the desired organ and the precision thereof.

Like the modification 1-2, another modification of the fourth embodiment may be made by adding a trace calculation unit 61.

The embodiments explained above are merely examples for carrying out the present invention. The present invention is not limited thereto, and it is within the scope of the present invention to make various kinds of modifications according to specifications and the like. Further, it is obvious from the above description that various kinds of other embodiments can be made within the scope of the present invention, as defined per the claims.

### EXPLANATIONS OF LETTERS OR NUMERALS

- 1: capsule endoscope system
- 2: capsule endoscope
- 2a: optical dome
- 2b: container
- 3: receiving device
- 4: antenna unit
- 5: image display apparatus
- 5a: cradle
- 5b: operation input device
- 10: subject
- 21: image-capturing unit
- 21a: image sensor
- 21b: optical system
- 22: illumination unit
- 23: circuit board
- 24, 32: signal processing unit
- 25, 33: memory
- 26: transmission unit
- 27: antenna
- 28, 37: battery
- 31: receiving unit
- 34, 51: interface (I/F) unit
- 35: operation unit
- 36: display unit
- 41a: receiving antenna
- 52: temporary storage unit
- 53: image processing unit
- 53a: basic processing unit
- 53b: red color detecting unit
- 53c: (tumorous) lesion detecting unit
- 53d: (vascular) lesion detecting unit 53d
- 53e: (bleeding) lesion detecting unit
- 54: position estimating unit
- 55: storage unit
- 56: processing setting unit
- 57: processing time estimating unit
- 58: examination information generating unit
- 59: display control unit
- 60: display unit
- 61: trace calculation unit
- 100, 130, 200, 300, 310, 400, 410: processing selection screen
- 101-107, 231-233: icon
- 108, 221, 234, 253, 263, 302: OK button
- 111: prediction processing time display field
- 112: "confirm" button
- 113: "return" button
- 120, 140: radiographic image interpretation screen
- 121: patient information region
- 122: diagnosis information region
- 123: main display region
- 124: reproduction operation button group
- 125: thumbnail region
- 126: time bar
- 127: position display region
- 128: person-shaped image
- 129: divided region
- 131, 311, 411: cursor
- 132, 312, 412: precision selection window
- 133, 254, 264, 313, 402, 413: radio button
- 201: selection button
- 202, 301: predicted time display region unit
- 203: processing time input field
- 210, 240: processing content table
- 220: processing display screen
- 222, 303: NO button
- 230: processing candidate display screen
- 235: instruction button
- 250: priority order setting screen
- 251, 261: processing name field
- 252: priority order input field
- 260: precision setting screen
- 262: precision input field
- 401: portion selection field

## Claims

1. A capsule endoscope system (1) comprising:
a capsule endoscope (2) that is configured to be introduced into a subject, so that the capsule endoscope (2) captures an in-vivo image and generates in-vivo image data representing the in-vivo image of the subject;
a receiving device (3) comprising multiple receiving antennas (41a - 41h) and configured to receive the in-vivo image data generated by the capsule endoscope (2) via wireless communication; and
an image display apparatus (5) configured to display an image based on the in-vivo image data obtained, via the receiving device (3), from the capsule endoscope (2), the image display apparatus (5) comprising:
an input receiving unit (51) configured to receive input of information to the image display apparatus (5);
a storage unit (52, 55) configured to store the in-vivo image data and information including received strength information about each of the receiving antennas (41a - 41h) when the in-vivo image is received and time information representing a time at which the in-vivo image is received, which are used for estimating a position of the capsule endoscope (2) in the subject, the information being associated with the in-vivo image data;
an image processing unit (53) configured to execute predetermined image processing on the in-vivo image data stored in the storage unit (52, 55);
a position estimating unit (54) configured to execute position estimating processing that estimates a position of the capsule endoscope (2) while the capsule endoscope (2) is capturing the in-vivo image, by using the received strength information and the time information included in the information related to the position stored in the storage unit (52, 55);
a display control unit (59);
a display unit (60) configured to display under control of the display control unit (59) a display screen including a selection screen (100) in which a user selects a position estimation level setting precision of the position estimating processing executed by the position estimating unit (54) and/or a type of the image processing executed by the image processing unit (53), wherein the selection screen (100) includes icons (101 - 104) representing a type of image processing, icons (105 - 107) representing the position estimation level, and an OK button (108);
a processing time estimating unit (57) configured to predict a processing time required in the image processing and/or the position estimating processing when the type of the image processing and the position estimation level desired by a user are selected and a selection signal of the OK button (108) is input in the selection screen (100); and
a processing setting unit (56) configured to control the image processing unit (53) and/or the position estimating unit (54) to execute processing,
wherein the display unit (60) is configured to display the type of the image processing selected in the selection screen (100) and a processing time confirmation screen (110) having a prediction processing time display field configured to display a prediction processing time predicted by the processing time estimating unit (57), and
when the input receiving unit (51) receives an input of an instruction for determining the selected type of the image processing displayed on the display unit (57) and the prediction processing time, the processing setting unit (56) controls the image processing unit (53) and/or the position estimating unit (54) to execute the determined processing.

2. The capsule endoscope system (1) according to claim 1, wherein the processing setting unit (56) is configured to control the image processing and the position estimating processing to be executed in parallel, and
the processing time estimating unit (57) is configured to estimate a processing time for completing the execution of the image processing and the position estimating processing in parallel.

3. The capsule endoscope system (1) according to claim 1, wherein the image processing unit (53) is configured to execute at least one of red color detecting processing and detecting processing of a predetermined lesion site.

4. The capsule endoscope system (1) according to claim 1, wherein the processing setting unit (56) is configured to set precision of the image processing executed by the image processing unit (53).

5. The capsule endoscope system (1) according to claim 1, further comprising a trace calculation unit (61) configured to calculate a trace of the capsule endoscope (2) in the subject, on the basis of execution result of the position estimating processing by the position estimating unit (54).

6. The capsule endoscope system (1) according to claim 1, wherein the input receiving unit (51) is configured to receive an input of a desired processing time or a desired finish time of the processing by the image processing unit (53) and/or the position estimating unit (54),
the processing setting unit (56) is configured to generate a processing content serving as a candidate of a position estimation level of the position estimating processing executed by the position estimating unit (54) and/or the content of the image processing executed by the image processing unit (53), on the basis of the desired processing time or the desired finish time, and
the display unit (60) is configured to display the processing content generated by the processing setting unit (56).

7. The capsule endoscope system (1) according to claim 6, wherein the processing setting unit (56) is configured to generate a processing content in which the image processing and/or the position estimating processing can be finished within a predetermined range from the desired processing time or the desired finish time.

8. The capsule endoscope system (1) according to claim 1, wherein the input receiving unit (51) is configured to receive an input of a desired processing time or a desired finish time for the processing by the image processing unit (53) and/or the position estimating unit (54),
the processing setting unit (56) is configured to generate a plurality of processing contents serving as candidates of position estimation levels of the position estimating processing executed by the position estimating unit (54) and/or the content of the image processing executed by the image processing unit (53), on the basis of the desired processing time or the desired finish time,
the display unit (60) is configured to display the plurality of processing contents, and
when the input receiving unit (51) receives an input of a selection signal for selecting one of the plurality of processing contents, the processing setting unit (56) is configured to set image processing included in the selected processing content as the processing executed by the image processing unit (53), and set a position estimating processing at a position estimation level included in the selected processing content as the processing executed by the position estimating unit (54).

9. The capsule endoscope system (1) according to claim 1, wherein the input receiving unit (51) is configured to receive an input of a desired processing time or a desired finish time of the processing by the image processing unit (53) and/or the position estimating unit (54), and receive an input of an order of priority of the image processing and/or the position estimating processing,
the processing setting unit (56) is configured to generate a processing content serving as a candidate of a position estimation level of the position estimating processing executed by the position estimating unit (54) and/or the content of the image processing executed by the image processing unit (53), on the basis of the desired processing time or the desired finish time and the order of priority, and
the display unit (60) is configured to display the processing content generated by the processing time estimating unit (57).

10. The capsule endoscope system (1) according to claim 1, wherein the processing time estimating unit (57) is configured to respectively predict individual processing times required by the position estimating processing at respective position estimation levels that can be executed by the position estimating unit (54) and various kinds of image processing that can be executed by the image processing unit (53), and
the display unit (60) is configured to display the individual processing times predicted by the processing time estimating unit (57) in association with the various kinds of image processing and the respective position estimation levels.

11. The capsule endoscope system (1) according to claim 1, wherein the input receiving unit (51) is configured to receive an input of selection information for selecting a portion of the subject on which the image processing and/or the position estimating processing are performed, and
the processing setting unit (56) is configured to set a range of the in-vivo image data to be subjected to the processing performed by the image processing unit (53) and/or the position estimation, on the basis of the selection information.

## Patentansprüche

1. Kapselendoskopsystem (1) mit:
einem Kapselendoskop (2), das dazu ausgebildet ist, in einen Patienten so eingeführt zu werden, dass das Kapselendoskop (2) ein In-vivo-Bild aufnimmt und In-vivo-Bilddaten erzeugt, die das In-vivo-Bild des Patienten darstellen;
einer Empfangsvorrichtung (3), die mehrere Empfangsantennen (41a - 41h) aufweist und dazu ausgebildet ist, die durch das Kapselendoskop (2) erzeugten In-vivo-Bilddaten über eine drahtlose Kommunikation zu empfangen; und
einer Bildanzeigevorrichtung (5), die dazu ausgebildet ist, ein Bild auf der Basis der vom Kapselendoskop (2) über die Empfangsvorrichtung (3) gewonnenen In-vivo-Bilddaten anzuzeigen, wobei die Bildanzeigevorrichtung (5) aufweist:
eine Eingabeempfangseinheit (51), die dazu ausgebildet ist, die in die Bildanzeigevorrichtung (5) eingegebenen Informationen zu empfangen;
eine Speichereinheit (52, 55), die zum Speichern der In-vivo-Bilddaten und von Informationen ausgebildet ist, die Empfangsstärkeinformationen über jede der Empfangsantennen (41a - 41h), wenn das In-vivo-Bild empfangen wird, und Zeitinformationen über die Zeit beinhalten, zu der das In-vivo-Bild empfangen wird, welche zum Abschätzen einer Position des Kapselendoskops (2) im Patienten verwendet werden, wobei die Informationen zu den In-vivo-Bilddaten gehören;
eine Bildverarbeitungseinheit (53), die dazu ausgebildet ist, eine vorgegebene Bildverarbeitung der in der Speichereinheit (52, 55) gespeicherten In-vivo-Bilddaten auszuführen;
eine Positionsschätzeinheit (54), die dazu ausgebildet ist, eine Positionsschätzverarbeitung auszuführen, die eine Position des Kapselendoskops (2) abschätzt, während das Kapselendoskop (2) das In-vivo-Bild aufnimmt, indem die Empfangsstärkeinformationen und die Zeitinformationen verwendet werden, die in den Informationen enthalten sind, die sich auf die in der Speichereinheit (52, 55) gespeicherte Position beziehen;
eine Anzeigesteuereinheit (59);
eine Anzeigeeinheit (60), die dazu ausgebildet ist, unter Steuerung der Anzeigesteuereinheit (59) eine Anzeigemaske anzuzeigen, die eine Auswahlmaske (100) enthält, in der ein Benutzer eine Positionsschätzgrad-Einstellgenauigkeit der durch die Positionsschätzeinheit (54) ausgeführten Positionsschätzverabeitung, und/oder eine Art der Bildverarbeitung auswählt, die durch die Bildverarbeitungseinheit (53) ausgeführt wird, wobei die Auswahlmaske (100) Symbolbilder (101 - 104), die eine Art von Bildverarbeitung darstellen, Symbolbilder (105 - 107), die den Positionsschätzgrad darstellen, und eine OK-Schaltfläche (108) enthält;
eine Verarbeitungszeit-Schätzeinheit (57), die dazu ausgebildet ist, eine Verarbeitungszeit vorauszuberechnen, die für die Bildverarbeitung und/oder für die Positionsschätzverarbeitung benötigt wird, wenn die von einem Benutzer gewünschte Art von Bildverarbeitung und der Positionsschätzgrad ausgewählt sind und ein Auswahlsignal der OK-Schaltfläche (108) in der Auswahlmaske (100) eingegeben ist; und
eine Verarbeitungseinstelleinheit (56), die dazu ausgebildet ist, die Bildverarbeitungseinheit (53) und/oder die Positionsschätzeinheit (54) zu steuern, um die Verarbeitung auszuführen,
wobei die Anzeigeeinheit (60) dazu ausgebildet ist, die in der Auswahlmaske (100) ausgewählte Art der Bildverarbeitung und eine Verarbeitungszeit-Bestätigungsmaske (110) anzuzeigen, die ein Prognoseverarbeitungszeit-Anzeigefeld besitzt, das dazu ausgebildet ist, eine durch die Verarbeitungszeit-Schätzeinheit (57) vorausberechnete Prognoseverarbeitungszeit anzuzeigen, und,
wenn die Eingabeempfangseinheit (51) eine Eingabe von einer Anweisung zum Bestimmen der ausgewählten Art der Bildverarbeitung, die auf der Anzeigeeinheit (57) angezeigt wird, und der Prognoseverarbeitungszeit empfängt, die Verarbeitungseinstelleinheit (56) die Bildverarbeitungseinheit (53) und/oder die Positionsschätzeinheit (54) steuert, um die bestimmte Verarbeitung auszuführen.

2. Kapselendoskopsystem (1) nach Anspruch 1, wobei die Verarbeitungseinstelleinheit (56) dazu ausgebildet ist, die Bildverarbeitung und die Positionsschätzverarbeitung so zu steuern, dass sie parallel ausgeführt werden, und
die Verarbeitungszeit-Schätzeinheit (57) dazu ausgebildet ist, eine Verarbeitungszeit zum Fertigstellen der parallelen Durchführung der Bildverarbeitung und der Positionsschätzverarbeitung abzuschätzen.

3. Kapselendoskopsystem (1) nach Anspruch 1, wobei die Bildverarbeitungseinheit (53) dazu ausgebildet ist, Verarbeitung von Rotlicht-Ermittlung und/oder die Verarbeitung der Ermittlung einer vorgegebenen Läsionsstelle auszuführen.

4. Kapselendoskopsystem (1) nach Anspruch 1, wobei die Verarbeitungseinstelleinheit (56) dazu ausgebildet ist, die Genauigkeit der durch die Bildverarbeitungseinheit (53) ausgeführten Bildverarbeitung einzustellen.

5. Kapselendoskopsystem (1) nach Anspruch 1, das des Weiteren eine Bahnberechnungseinheit (61) aufweist, die dazu ausgebildet ist, eine Bahn des Kapselendoskops (2) im Patienten auf der Basis des Ausführungsergebnisses der Positionsschätzverarbeitung durch die Positionsschätzeinheit (54) zu berechnen.

6. Kapselendoskopsystem (1) nach Anspruch 1, wobei die Eingabeempfangseinheit (51) dazu ausgebildet ist, eine Eingabe einer gewünschten Verarbeitungszeit oder einer gewünschten Beendigungszeit der Verarbeitung durch die Bildverarbeitungseinheit (53) und/oder die Positionsschätzeinheit (54) zu empfangen,
die Verarbeitungseinstelleinheit (56) dazu ausgebildet ist, auf der Basis der gewünschten Verarbeitungszeit oder der gewünschten Beendigungszeit einen Verarbeitungsinhalt, der als ein Anwärter eines Positionsschätzgrads der durch die Positionsschätzeinheit (54) ausgeführten Positionsschätzverarbeitung dient, und/oder den Inhalt der Bildverarbeitung zu erzeugen, die durch die Bildverarbeitungseinheit (53) ausgeführt wird, und
die Anzeigeeinheit (60) dazu ausgebildet ist, den durch die Verarbeitungseinstelleinheit (56) erzeugten Verarbeitungsinhalt anzuzeigen.

7. Kapselendoskopsystem (1) nach Anspruch 6, wobei die Verarbeitungseinstelleinheit (56) dazu ausgebildet ist, einen Verarbeitungsinhalt zu erzeugen, bei dem die Bildverarbeitung und/oder die Positionsschätzverarbeitung innerhalb eines vorgegebenen Bereichs der gewünschten Verarbeitungszeit oder der gewünschten Beendigungszeit fertiggestellt werden kann.

8. Kapselendoskopsystem (1) nach Anspruch 1, wobei die Eingabeempfangseinheit (51) dazu ausgebildet ist, eine Eingabe einer gewünschten Verarbeitungszeit oder einer gewünschten Beendigungszeit für die Verarbeitung durch die Bildverarbeitungseinheit (53) und/oder die Positionsschätzeinheit (54) zu empfangen,
die Verarbeitungseinstelleinheit (56) dazu ausgebildet ist, auf der Basis der gewünschten Verarbeitungszeit oder der gewünschten Beendigungszeit eine Mehrzahl von Verarbeitungsinhalten, die als Anwärter von Positionsschätzgraden der durch die Positionsschätzeinheit (54) ausgeführten Positionsschätzverarbeitung dienen, und/oder den Inhalt der Bildverarbeitung zu erzeugen, die durch die Bildverarbeitungseinheit (53) ausgeführt wird,
die Anzeigeeinheit (60) dazu ausgebildet ist, die Mehrzahl von Verarbeitungsinhalten anzuzeigen, und,
wenn die Eingabeempfangseinheit (51) eine Eingabe eines Auswahlsignals zum Auswählen eines aus der Mehrzahl von Verarbeitungsinhalten empfängt, die Verarbeitungseinstelleinheit (56) dazu ausgebildet ist, die Bildverarbeitung, die in dem gewählten Verarbeitungsinhalt enthalten ist, als die Verarbeitung einzustellen, die durch die Bildverarbeitungseinheit (53) ausgeführt wird, und eine Positionsschätzverarbeitung bei einem Positionsschätzgrad einzustellen, der in dem gewählten Verarbeitungsinhalt als die Verarbeitung enthalten ist, die durch die Positionsschätzeinheit (54) ausgeführt wird.

9. Kapselendoskopsystem (1) nach Anspruch 1, wobei die Eingabeempfangseinheit (51) dazu ausgebildet ist, eine Eingabe einer gewünschten Verarbeitungszeit oder einer gewünschten Beendigungszeit der Verarbeitung durch die Bildverarbeitungseinheit (53) und/oder die Positionsschätzeinheit (54) zu empfangen, und eine Eingabe einer Prioritätenfolge der Bildverarbeitung und/oder der Positionsschätzverarbeitung zu empfangen,
die Verarbeitungseinstelleinheit (56) dazu ausgebildet ist, auf der Basis der gewünschten Verarbeitungszeit oder der gewünschten Beendigungszeit und der Prioritätenfolge einen Verarbeitungsinhalt, der als ein Anwärter eines Positionsschätzgrads der durch die Positionsschätzeinheit (54) ausgeführten Positionsschätzverarbeitung dient, und/oder den Inhalt der Bildverarbeitung zu erzeugen, die durch die Bildverarbeitungseinheit (53) ausgeführt wird, und
die Anzeigeeinheit (60) dazu ausgebildet ist, den durch die Verarbeitungszeit-Schätzeinheit (57) erzeugten Verarbeitungsinhalt anzuzeigen.

10. Kapselendoskopsystem (1) nach Anspruch 1, wobei die Verarbeitungszeit-Schätzeinheit (57) dazu ausgebildet ist, jeweils einzelne Verarbeitungszeiten vorherzusagen, die durch die Positionsschätzverarbeitung bei entsprechenden Positionsschätzgraden, die durch die Positionsschätzeinheit (54) ausgeführt werden können, und durch verschiedene Arten von Bildverarbeitung, die durch die Bildverarbeitungseinheit (53) ausgeführt werden können, benötigt werden, und
die Anzeigeeinheit (60) dazu ausgebildet ist, die einzelnen Verarbeitungszeiten anzuzeigen, die durch die Verarbeitungszeit-Schätzeinheit (57) in Verbindung mit den verschiedenen Arten von Bildverarbeitung und den entsprechenden Positionsschätzgraden vorhergesagt werden.

11. Kapselendoskopsystem (1) nach Anspruch 1, wobei die Eingabeempfangseinheit (51) dazu ausgebildet ist, eine Eingabe einer Auswahlinformation zum Auswählen eines Bereiches des Patienten zu empfangen, an dem die Bildverarbeitung und/oder die Positionsschätzverarbeitung durchgeführt werden, und
die Verarbeitungseinstelleinheit (56) dazu ausgebildet ist, auf der Basis der Auswahlinformation einen Bereich der In-vivo-Bilddaten einzustellen, welcher der Verarbeitung, die durch die Bildverarbeitungseinheit (53) ausgeführt wird, und/oder der Positionsschätzung zu unterziehen ist.

## Revendications

1. Système d'endoscope de type capsule (1) comprenant :
un endoscope de type capsule (2) qui est configuré pour être introduit dans un sujet, de sorte que l'endoscope de type capsule (2) capture une image in vivo et génère des données d'image in vivo représentant l'image in vivo du sujet ;
un dispositif de réception (3) comprenant de multiples antennes de réception (41a à 41h) et configuré pour recevoir les données d'image in vivo générées par l'endoscope de type capsule (2) via une communication sans fil ; et
un appareil d'affichage d'images (5) configuré pour afficher une image sur la base des données d'image in vivo obtenues, via le dispositif de réception (3), depuis l'endoscope de type capsule (2), l'appareil d'affichage d'images (5) comprenant :
une unité de réception d'entrée (51) configurée pour recevoir une entrée d'informations vers l'appareil d'affichage d'images (5) ;
une unité de mémoire (52, 55) configurée pour mémoriser les données d'image in vivo et des informations incluant des informations de force reçues concernant chacune des antennes de réception (41a à 41h) lorsque l'image in vivo est reçue et les informations de temps représentant un moment auquel l'image in vivo est reçue, qui sont utilisées pour estimer une position de l'endoscope de type capsule (2) dans le sujet, les informations étant associées aux données d'image in vivo ;
une unité de traitement d'image (53) configurée pour exécuter un traitement d'image prédéterminé sur les données d'image in vivo mémorisées dans l'unité de mémoire (52, 55) ;
une unité d'estimation de position (54) configurée pour exécuter un traitement d'estimation de position qui estime une position de l'endoscope de type capsule (2) alors que l'endoscope de type capsule (2) est en train de capturer l'image in vivo, en utilisant les informations de force reçues et les informations de temps contenues dans les informations se rapportant à la position mémorisée dans l'unité de mémoire (52, 55) ;
une unité de commande d'affichage (59) ;
une unité d'affichage (60) configurée pour afficher sous la commande de l'unité de commande d'affichage (59) un écran d'affichage incluant un écran de sélection (100) dans lequel un utilisateur sélectionne une précision de réglage de niveau d'estimation de position du traitement d'estimation de position exécuté par l'unité d'estimation de position (54) et/ou un type de traitement d'image exécuté par l'unité de traitement d'image (53), dans laquelle l'écran de sélection (100) comprend des icônes (101 à 104) représentant un type de traitement d'image, des icônes (105 à 107) représentant le niveau d'estimation de position, et un bouton OK (108) ;
une unité d'estimation de temps de traitement (57) configurée pour prédire un temps de traitement requis dans le traitement d'image et/ou le traitement d'estimation de position lorsque le type de traitement d'image et le niveau d'estimation de position souhaités par un utilisateur sont sélectionnés et un signal de sélection du bouton OK (108) est délivré en entrée dans l'écran de sélection (100) ; et
une unité de fixation de traitement (56) configurée pour commander l'unité de traitement d'image (53) et/ou l'unité d'estimation de position (54) pour exécuter le traitement,
dans lequel l'unité d'affichage (60) est configurée pour afficher le type de traitement d'image sélectionné dans l'écran de sélection (100) et un écran de confirmation de temps de traitement (110) présentant un champ d'affichage de temps de traitement de prédiction configuré pour afficher un temps de traitement de prédiction prédit par l'unité d'estimation du temps de traitement (57), et
lorsque l'unité de réception d'entrée (51) reçoit une entrée d'une instruction destinée à déterminer le type de traitement d'image sélectionné affiché sur l'unité d'affichage (57) et le temps de traitement de prédiction, l'unité de fixation de traitement (56) commande l'unité de traitement d'image (53) et/ou l'unité d'estimation de position (54) pour exécuter le traitement déterminé.

2. Système d'endoscope de type capsule (1) selon la revendication 1, dans lequel l'unité de fixation de traitement (56) est configurée pour commander le traitement d'image et le traitement d'estimation de position devant être exécutés en parallèle, et
l'unité d'estimation du temps de traitement (57) est configurée pour estimer un temps de traitement pour terminer l'exécution du traitement d'image et du traitement d'estimation de position en parallèle.

3. Système d'endoscope de type capsule (1) selon la revendication 1, dans lequel l'unité de traitement d'image (53) est configurée pour exécuter au moins l'un parmi un traitement de détection de couleur rouge et un traitement de détection d'un site de lésion prédéterminé.

4. Système d'endoscope de type capsule (1) selon la revendication 1, dans lequel l'unité de fixation de traitement (56) est configurée pour fixer une précision du traitement d'image exécuté par l'unité de traitement d'image (53).

5. Système d'endoscope de type capsule (1) selon la revendication 1, comprenant en outre une unité de calcul de trace (61) configuré pour calculer une trace de l'endoscope de type capsule (2) dans le sujet, sur la base d'un résultat d'exécution du traitement d'estimation de position par l'unité d'estimation de position (54).

6. Système d'endoscope de type capsule (1) selon la revendication 1, dans lequel l'unité de réception d'entrée (51) est configurée pour recevoir une entrée d'un temps de traitement souhaité ou d'un moment de fin souhaité du traitement par l'unité de traitement d'images (53) et/ou l'unité d'estimation de position (54),
l'unité de fixation de traitement (56) est configurée pour générer un contenu de traitement servant de candidat d'un niveau d'estimation de position du traitement d'estimation de position exécuté par l'unité d'estimation de position (54) et/ou le contenu du traitement d'image exécuté par l'unité de traitement d'image (53), sur la base du temps de traitement souhaité ou du moment de fin souhaité, et
l'unité d'affichage (60) est configurée pour afficher le contenu de traitement généré par l'unité de fixation de traitement (56).

7. Système d'endoscope de type capsule (1) selon la revendication 6, dans lequel l'unité de fixation de traitement (56) est configurée pour générer un contenu de traitement dans lequel le traitement d'image et/ou le traitement d'estimation de position peuvent être terminés à l'intérieur d'une plage prédéterminée à partir du temps de traitement souhaité ou du moment de fin souhaité.

8. Système d'endoscope de type capsule (1) selon la revendication 1, dans lequel l'unité de réception d'entrée (51) est configurée pour recevoir une entrée d'un temps de traitement souhaité ou d'un moment de fin souhaité pour le traitement par l'unité de traitement d'image (53) et/ou l'unité d'estimation de position (54),
l'unité de fixation de traitement (56) est configurée pour générer une pluralité de contenus de traitement servant de candidats des niveaux d'estimation de position du traitement d'estimation de position exécuté par l'unité d'estimation de position (54) et/ou le contenu du traitement d'image exécuté par l'unité de traitement d'image (53), sur la base du temps de traitement souhaité ou du moment de fin souhaité,
l'unité d'affichage (60) est configurée pour afficher la pluralité de contenus de traitement, et
lorsque l'unité de réception d'entrée (51) reçoit une entrée d'un signal de sélection destiné à sélectionner l'un parmi la pluralité de contenus de traitement, l'unité de fixation de traitement (56) est configurée pour fixer un traitement d'image inclus dans le contenu de traitement sélectionné en tant que traitement exécuté par l'unité de traitement d'image (53), et fixer un traitement d'estimation de position à un niveau d'estimation de position inclus dans le contenu de traitement sélectionné en tant que traitement exécuté par l'unité d'estimation de position (54).

9. Système d'endoscope de type capsule (1) selon la revendication 1, dans lequel l'unité de réception d'entrée (51) est configurée pour recevoir une entrée d'un temps de traitement souhaité ou d'un moment de fin souhaité du traitement par l'unité de traitement d'image (53) et/ou l'unité d'estimation de position (54), et recevoir une entrée d'un ordre de priorité du traitement d'image et/ou du traitement d'estimation de position,
l'unité de fixation de traitement (56) est configurée pour générer un contenu de traitement servant de candidat d'un niveau d'estimation de position du traitement d'estimation de position exécuté par l'unité d'estimation de position (54) et/ou le contenu du traitement d'image exécuté par l'unité de traitement d'image (53), sur la base du temps de traitement souhaité ou du moment de fin souhaité et de l'ordre de priorité, et
l'unité d'affichage (60) est configurée pour afficher le contenu de traitement généré par l'unité d'estimation de temps de traitement (57).

10. Système d'endoscope de type capsule (1) selon la revendication 1, dans lequel l'unité d'estimation de temps de traitement (57) est configurée pour prédire respectivement des temps de traitement individuels requis par le traitement d'estimation de position à des niveaux d'estimation de position respectifs qui peuvent être exécuté par l'unité d'estimation de position (54) et divers types de traitements d'image qui peuvent être exécutés par l'unité de traitement d'image (53), et
l'unité d'affichage (60) est configurée pour afficher les temps de traitement individuels prédits par l'unité d'estimation du temps de traitement (57) en association avec les divers types de traitements d'image et les niveaux d'estimation de position respectifs.

11. Système d'endoscope de type capsule (1) selon la revendication 1, dans lequel l'unité de réception d'entrée (51) est configurée pour recevoir une entrée d'informations de sélection destinée à sélectionner une partie du sujet sur laquelle le traitement d'image et/ou le traitement d'estimation de position sont exécutés, et
l'unité de fixation de traitement (56) est configurée pour fixer une plage des données d'image in vivo devant être soumises au traitement réalisé par l'unité de traitement d'image (53) et/ou l'estimation de position, sur la base des informations de sélection.
